# EUROPEAN PATENT APPLICATION

(11) **EP 3 290 116 A1**
(43) Date of publication of application: **07.03.2018**
(21) Application number: 17187033.0
(22) Date of filing: 21.08.2017
(51) Int. Cl.: B01L 3/00

(54) **CARTRIDGE FOR SAMPLE ANALYSIS, PRODUCTION METHOD THEREOF, AND USE THEREOF**

(30) Priority: 30.08.2016 JP 2016168313
(71) Applicant: SYSMEX CORPORATION, Kobe-shi Hyogo 651-0073 (JP)
(72) Inventor: HORII, Kazuyoshi, Hyogo, 651-0073 (JP); NODA, Kenta, Hyogo, 651-0073 (JP)
(74) Representative: Hoffmann Eitle

(57) **Abstract**

Disclosed is a cartridge for sample analysis, including: a cartridge base body having, formed therein, a chamber configured to store a liquid containing a test substance, and a storage portion which is connectable with the chamber and is configured to store a liquid reagent to be supplied to the chamber; carrier particles immobilized onto an inner wall of the chamber; a first liquid reagent stored in the storage portion; and a first capture substance that specifically binds to the test substance. The carrier particles are immobilized onto the inner wall by means of a solid mixture containing sugar and protein.

## Description

### FIELD OF THE INVENTION

The present invention relates to a cartridge for sample analysis and a production method thereof. Further, the present invention relates to a method for detecting a test substance by using the cartridge for sample analysis. Still further, the present invention relates to a method for immobilizing carrier particles.

### BACKGROUND

As a method for detecting a test substance in a sample, a method using a microfluidic device has been known. Examples of the microfluidic device include a chip, a cartridge, and the like in which fine flow paths and reaction chambers are formed. A microfluidic device used for sample analysis is provided with: a reagent containing a capture substance that can bind to a test substance; and a solid phase carrier, in advance. In a detection method using the microfluidic device, a series of operations, such as mixing of a sample and a reagent, reaction between a test substance and a capture substance, separation of a complex containing the test substance, and detection of the test substance, are performed in the device.

US Patent Publication No. 8951417 discloses a microfluidic device provided with magnetic particles, which can be used for detection of a test substance. This literature discloses a method for, using a disk-shaped microfluidic device having a plurality of chambers, transferring magnetic particles from one chamber to a next chamber by means of a magnetic force and a centrifugal force caused by rotation of the device.

The present inventors attempted to produce a microfluidic device provided with carrier particles. Specifically, the present inventors produced a cartridge in which a container for containing liquid is formed, and stored a suspension of carrier particles in the container formed in the cartridge. However, deposition of the carrier particles occurred while the cartridge was preserved, and the container was clogged with the carrier particles when the cartridge was used. In order to solve this problem, the present inventors attempted to dry and immobilize the carrier particles in the cartridge.

Meanwhile, the present inventors found that immobilization strength and dispersibility of the carrier particles need to be improved when the carrier particles are dried and immobilized in the cartridge. For example, during transport of a cartridge as a product, if carrier particles immobilized to a predetermined portion of the cartridge are separated from the portion due to vibration, impact, or the like, the separated carrier particles may enter another portion of the cartridge. Such carrier particles may have adverse effect on test-substance detecting performance. In order to accurately detect a test substance, immobilized carrier particles are desired to be easily dispersed into a solution containing the test substance. That is, both the two conditions, i.e., high immobilization strength of carrier particles and excellent dispersibility thereof, need to be satisfied.

Considering the circumstances described above, an object of the present invention is to provide: a cartridge for sample analysis, in which carrier particles are dried and immobilized so as to be firmly immobilized and be easily dispersible into a liquid; and a method for detecting a test substance by using the cartridge.

### SUMMARY OF THE INVENTION

(1) A first aspect of the present invention provides a cartridge for sample analysis. This cartridge for sample analysis includes: a cartridge base body having, formed therein, a chamber configured to store a liquid containing a test substance, and a storage portion which is connectable with the chamber and is configured to store a liquid reagent to be supplied to the chamber; carrier particles held onto an inner wall of the chamber; a first liquid reagent stored in the storage portion; and a first capture substance that specifically binds to the test substance. Further, in the cartridge for sample analysis, the carrier particles are immobilized onto the inner wall of the chamber by means of a solid mixture containing sugar and protein.
(2) The cartridge of (1), wherein a mass ratio of the sugar to the protein in the solid mixture is not less than 0.1 and not greater than 100.
(3) The cartridge of (2), wherein the mass ratio of the sugar to the protein in the solid mixture is not less than 0.5 and not greater than 30.
(4) The cartridge of any one of (1) to (3), wherein a mass ratio of the carrier particles to the protein contained in the solid mixture is not less than 0.05 and not greater than 10.
(5) The cartridge of any one of (1) to (4), wherein
   the carrier particles are obtained by drying, on the inner wall of the chamber, a suspension of the carrier particles which is obtained by dispersing the carrier particles into the solution containing sugar and protein, and
   a concentration of the protein in the suspension of the carrier particles is not less than 0.1 mass% and not greater than 20 mass%.
(6) The cartridge of (5), wherein a concentration of the sugar in the suspension of the carrier particles is not less than 0.5 mass% and not greater than 30 mass%.
(7) The cartridge of any one of (1) to (6), wherein the first capture substance is contained in the first liquid reagent.
(8) The cartridge of any one of (1) to (6), wherein the first capture substance is immobilized to surfaces of the carrier particles.
(9) The cartridge of any one of (1) to (8), wherein the first capture substance is an antibody or an antigen that specifically binds to the test substance.
(10) The cartridge of any one of (1) to (9), wherein the carrier particles are magnetic particles.
(11) The cartridge of any one of (1) to (10), wherein the sugar contained in the solid mixture is at least one selected from among a monosaccharide and a disaccharide.
(12) The cartridge of (11), wherein the sugar contained in the solid mixture is at least one selected from among sucrose, trehalose, glucose, lactose, fructose, maltose, and galactose.
(13) The cartridge of any one of (1) to (12), wherein the protein contained in the solid mixture is at least one selected from among proteins having function of stabilizing the carrier particles and the first capture substance.
(14) The cartridge of (13), wherein the protein contained in the solid mixture is at least one selected from among albumin, crystallin, casein, normal serum protein, collagen, gelatin, Gelysate, skim milk, lactic acid ferment, and decomposition products thereof.
(15) The cartridge of any one of (1) to (10), wherein the sugar contained in the solid mixture is sucrose, and the protein contained in the solid mixture is bovine serum albumin.
(16) The cartridge of any one of (1) to (15), wherein the chamber to which the carrier particles are immobilized is made of a material selected from among cycloolefin polymer, polymethyl methacrylate, cycloolefin copolymer, polypropylene, polyethylene, polystyrene, polycarbonate, acrylonitrile-butadiene-styrene copolymer, and glass.
(17) The cartridge of any one of (1) to (16), wherein the cartridge has a plate shape.
(18) The cartridge of any one of (1) to (17), further comprising a second liquid reagent containing a second capture substance that specifically binds to the test substance,
   the second liquid reagent being stored in a storage portion different from the storage portion where the first liquid reagent is stored.
(19) A second aspect of the present invention provides a method for detecting a test substance by using a cartridge for sample analysis. This method includes: bringing carrier particles immobilized to an inner wall of a chamber formed in the cartridge for sample analysis, a sample containing the test substance, a first capture substance that specifically binds to the test substance, and a first liquid reagent, into contact with each other in the chamber; dispersing, by agitation, the carrier particles into a liquid mixture containing the test substance, the first capture substance, and the first liquid reagent; forming a complex containing the test substance and the first capture substance, on the dispersed carrier particles; and detecting the test substance contained in the complex. In the cartridge for sample analysis used in this method, the carrier particles are immobilized onto the inner wall by means of a solid mixture containing sugar and protein.
(20) The method of (19), wherein a mass ratio of the sugar to the protein in the solid mixture is not less than 0.1 and not greater than 100.
(21) The method of (20), wherein the mass ratio of the sugar to the protein in the solid mixture is not less than 0.5 and not greater than 30.
(22) The method of any one of (19) to (21), wherein a mass ratio of the carrier particles to the protein contained in the solid mixture is not less than 0.05 and not greater than 10 in the cartridge for sample analysis.
(23) The method of any one of (19) to (22), wherein the first liquid reagent is a solution containing the first capture substance that specifically binds to the test substance.
(24) The method of any one of (19) to (23), wherein the carrier particles are particles having surfaces to which the first capture substance that specifically binds to the test substance is immobilized.
(25) The method of any one of (19) to (24), wherein the first capture substance is an antibody or an antigen that specifically binds to the test substance.
(26) The method of any one of (19) to (25), wherein the carrier particles are magnetic particles.
(27) The method of any one of (19) to (26), wherein the sugar contained in the solid mixture is at least one selected from among a monosaccharide and a disaccharide.
(28) The method of (27), wherein the sugar contained in the solid mixture is at least one selected from among sucrose, trehalose, glucose, lactose, fructose, maltose, and galactose.
(29) The method of any one of (19) to (28), wherein the protein contained in the solid mixture is at least one selected from among proteins having function of stabilizing the carrier particles and the first capture substance.
(30) The method of (29), wherein the protein contained in the solid mixture is at least one selected from among albumin, crystallin, casein, normal serum protein, collagen, gelatin, Gelysate, skim milk, lactic acid ferment, and decomposition products thereof.
(31) The method of any one of (19) to (26), wherein the sugar contained in the solid mixture is sucrose, and the protein contained in the solid mixture is bovine serum albumin.
(32) The method of any one of (19) to (31), wherein the sample is blood, plasma, or serum.
(33) The method of any one of (19) to (32), further comprising, between the forming of the complex and the detecting of the complex, adding a second liquid reagent containing a second capture substance that specifically binds to the test substance, and forming a complex containing the test substance, the first capture substance, and the second capture substance, on the dispersed carrier particles.
(34) The method of (33), wherein
   the second capture substance is a labeled antibody or a labeled antigen that specifically binds to the test substance, and
   the test substance is detected on the basis of labeling of the labeled antibody or the labeled antigen contained in the complex.
(35) The method of any one of (19) to (34), wherein the agitation is performed by applying at least one of a centrifugal force and an inertial force to the cartridge, and causing magnitudes of the applied centrifugal force and inertial force to change with time.
(36) A third aspect of the present invention provides a method for producing a cartridge for sample analysis. This method includes: dispersing carrier particles into a solution containing sugar and protein to obtain a suspension of the carrier particles; supplying the suspension to a region, on a substrate, where a chamber for storing therein a liquid containing a test substance is to be formed; drying the supplied suspension to immobilize the carrier particles to the region on the substrate; and forming a chamber that encloses the dried and immobilized carrier particles.
(37) A fourth aspect of the present invention provides a method for immobilizing carrier particles onto a solid phase. This method includes: dispersing the carrier particles into a solution containing sugar and protein to obtain a suspension of the carrier particles; supplying the suspension onto a solid phase; and drying the supplied suspension to immobilize the carrier particles onto the solid phase.
(38) The method of (36) or (37), wherein a mass ratio of the sugar to the protein in the suspension is not less than 0.1 and not greater than 100.
(39) The method of any one of (36) to (38), wherein a concentration of the protein in the suspension of the carrier particles is not less than 0.1 mass% and not greater than 20 mass%.
(40) The method of (39), wherein a concentration of the sugar in the suspension of the carrier particles is not less than 0.5 mass% and not greater than 30 mass%.

According to the present invention, it is possible to provide a cartridge for sample analysis, in which carrier particles are dried and immobilized such that the carrier particles bear vibration or impact during transport of the cartridge, and the carrier particles are easily dispersed into a liquid when the cartridge is used. Further, the present invention enables detection of a test substance in a sample by using such a cartridge for sample analysis.

### BRIEF DESCRIPTION OF THE DRAWINGS

FIG. 1 shows a photograph (left) of magnetic particles immobilized to an inner wall of a chamber, and a photograph (right) of the magnetic particles after a centrifugal force of 500 G has been applied thereto by centrifugation at 3000 rpm for 30 seconds;
FIG. 2A shows a photograph (left) of magnetic particles before agitation by centrifugation is performed, and a photograph (right) of the magnetic particles after agitation by centrifugation has been performed;
FIG. 2B shows a photograph (left) of magnetic particles before agitation by centrifugation is performed, and a photograph (right) of the magnetic particles after agitation by centrifugation has been performed;
FIG. 3A is a graph showing a ratio (%) of a signal obtained by measurement using dried and immobilized magnetic particles, to a signal obtained by measurement using a commercially available liquid reagent;
FIG. 3B is a graph showing a ratio (%) of a signal obtained by measurement using dried and immobilized magnetic particles, to a signal obtained by measurement using a commercially available liquid reagent;
FIG. 4A is a schematic diagram showing an example of an external structure of an analyzer;
FIG. 4B is a schematic diagram showing an example of the structure of a cartridge for sample analysis, as viewed from above;
FIG. 5 shows the structures of a mounting member, a magnet, a movement mechanism, a detection unit, and a housing of the analyzer, as viewed from diagonally above;
FIG. 6 is a schematic diagram showing a state where light generated from a chamber is received by a photodetector, as viewed from the side thereof;
FIG. 7 shows the structure of a body of the analyzer as viewed from above, and the structure of a lid of the analyzer as viewed from diagonally below;
FIG. 8 is a cross-sectional view of the analyzer which is cut in a vertical plane that passes a rotation shaft;
FIG. 9 is a flowchart showing an operation of the analyzer;
FIG. 10 is a flowchart showing an operation of the analyzer when a complex is transferred between adjacent chambers;
FIG. 11A schematically shows a manner of transferring a complex between adjacent chambers;
FIG. 11B schematically shows a manner of transferring the complex between adjacent chambers;
FIG. 11C schematically shows a manner of transferring the complex between adjacent chambers;
FIG. 12A schematically shows a manner of transferring the complex between adjacent chambers;
FIG. 12B schematically shows a manner of transferring the complex between adjacent chambers;
FIG. 12C schematically shows a manner of transferring the complex between adjacent chambers; and
FIG. 13 is a schematic diagram showing an example of the structure of a cartridge for sample analysis, as viewed from above.

### DETAILED DESCRIPTION OF THE PREFERRED EMBODIMENTS

### [1. Cartridge for sample analysis]

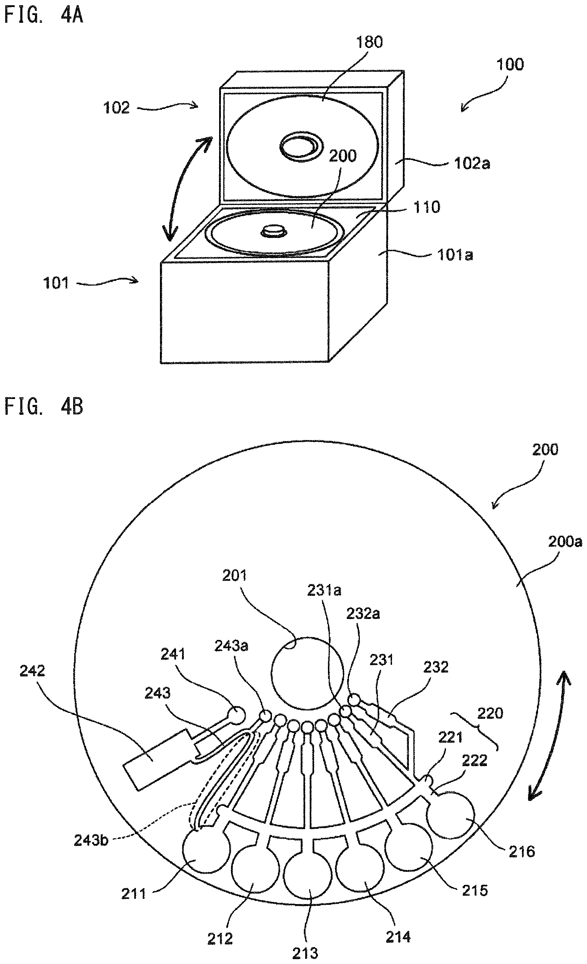

A cartridge for sample analysis (hereinafter also simply referred to as "cartridge") according to the present embodiment is a microfluidic device in which a solid phase carrier and a detection reagent, which are necessary for detection of a test substance in a sample, are appropriately arranged. Detection of the test substance is realized by infusing the sample containing the test substance into the cartridge of the present embodiment and treating the sample in a predetermined process. The predetermined process is preferably executed by using an analyzer as shown in FIG. 4A, which is dedicated to the cartridge of the present embodiment. The analyzer 100 shown in FIG. 4A will be described later.

The sample to be analyzed by using the cartridge of the present embodiment is not particularly limited as long as the sample contains the test substance. Examples of the sample include: biological samples such as blood, plasma, serum, lymph fluid, and lysates of cells or tissues; excrements such as urine and feces; and environmental samples such as river water, sea water, and soil. The cartridge of the present embodiment is suitable for detection of test substances in blood, plasma, and serum.

The kind of the test substance is not particularly limited as long as a capture substance that specifically binds to the test substance exists or such a capture substance can be produced. When an antibody is used as a capture substance, any substance having antigenicity can be a test substance. Examples of the test substance include proteins, peptides, nucleic acids, bioactive substances, vesicles, bacteria, viruses, haptens, therapeutic agents, and metabolites of therapeutic agents. An antibody can also be a test substance. Examples of the proteins include proteins that exist in nature, and non-natural proteins such as recombinant proteins. Examples of the peptides include polypeptides having a large number of amino acid residues, and oligopeptides having a less number of amino acid residues, such as dipeptides and tripeptides. Examples of the nucleic acids include nucleic acids that exist in nature, and artificially synthesized nucleic acids such as nucleic acid analogue. Examples of the polysaccharides include sugar chains that exist at a surface of a cell or a protein, and lipopolysaccharides that are outer membrane components of bacteria. Examples of the bioactive substances include, but are not particularly limited to, cell growth factors, differentiation inducing factors, cell adhesion factors, enzymes, cytokines, hormones, sugar chains, and lipids. The vesicles are not particularly limited as long as they are formed of membranes. The vesicles may contain liquid phases therein. Examples of the vesicles include: extracellular vesicles such as exosomes, microvesicles, and apoptotic bodies; and artificial vesicles such as liposomes.

The cartridge of the present embodiment is provided with at least a cartridge base body, carrier particles, a first liquid reagent, and a first capture substance that specifically binds to the test substance.

The cartridge base body (hereinafter also simply referred to as "base body") is a member that serves as: a storage container for storing therein the carrier particles and the liquid reagent, which are necessary for detection of a test substance, until they are used; and a reaction container in which reaction between a sample and a reagent is performed. In the present embodiment, a chamber and a storage portion are formed in the base body. The chamber stores therein a liquid containing a test substance. The storage portion is connectable with the chamber and stores therein a liquid reagent to be supplied to the chamber. The chamber is a portion, of the base body, corresponding to the reaction container, and the storage portion is a portion, of the base body, corresponding to the storage container. In the base body, the chamber and the storage portion are connected to each other via a flow path (channel). The thickness of the base body is not particularly limited, and may be not less than 0.2 mm and not greater than 10 mm, for example. The volumes of the chamber and the storage portion are not particularly limited, and may be not less than 0.1 µL and not greater than 500 µL, for example. The inner diameter or the width of the channel is not particularly limited, and may be not less than 0.1 µm and not greater than 5000 µm, for example.

In the present embodiment, a plurality of chambers may be formed in the base body. A plurality of storage portions connectable with the chambers may be formed in the base body. In this case, the number of the chambers may be equal to or different from the number of the storage portions. When a plurality of chambers are formed in the base body, a channel as a flow path for connecting the chambers to each other is preferably formed in the base body. In addition, an opening, through which the sample containing the test substance is infused from the outside, is preferably formed in the base body.

As the material of the cartridge base body, the same material as a solid phase generally used for immunological measurement can be suitably used. Examples of the material include cycloolefin polymer (COP), polymethyl methacrylate (PMMA), cycloolefin copolymer, polypropylene, polyethylene, polystyrene, polycarbonate, acrylonitrile-butadiene-styrene copolymer, and glass. One of these materials may be used solely, or two or more of these materials may be used in combination. For example, the chamber may be formed of one material selected from among the above materials, and the remaining portions, such as the channel and the storage portion, may be formed of materials different from the material of the chamber. In the present embodiment, the material of the cartridge base body is preferably a material that allows light to pass therethrough.

In the present embodiment, the base body can be obtained by forming the chamber and the storage portion in a substrate which is an essential member of the base body. Further, the channel and the opening are preferably formed in the substrate. That is, the substrate is a member in which the chamber, the storage portion, the channel, and the opening are formed. The substrate is preferably a plate-shaped member made of the same material as the base body. For example, the base body can be obtained by forming recesses to be the chamber, the storage portion, the channel, and the opening at a surface of the substrate, and bonding a film to the substrate so as to cover the entire surface of the substrate. However, with reference to FIG. 4B, no film is bonded to an upper surface at positions where an opening 241 and seals 231 a and 232a are present. When the recesses formed in the substrate are covered with the film, the recesses become the chamber, the storage portion, the channel, and the opening.

Alternatively, the base body can be obtained by bonding a substrate in which recesses to be the chamber, the storage portion, and the channel and a hole to be the opening are formed, to another substrate in which recesses to be the chamber, the storage portion, the channel, and the opening are formed in a symmetrical manner with respect to those of the above-mentioned substrate. The hole to be the opening may be formed in one of the substrates. Still alternatively, the base body can be obtained by mounting components forming the chamber, the storage portion, and the channel on a substrate. In this case, the mounted components are preferably fixed onto the substrate by means of an adhesive agent or a fusing technique.

The opening is an inlet through which the sample containing the test substance is infused from the outside into the cartridge. The opening is connected to one of chambers via a flow path. According to the kind of the sample, a sample storage portion for temporarily storing the sample therein may be formed in the cartridge base body between the opening and the chamber to which the opening is connected. For example, when the sample is a liquid sample containing the test substance and insoluble contaminants, the sample storage portion can be used as a place (separator) for separating the liquid sample into a supernatant containing the test substance, and the insoluble contaminants.

In the base body, arrangement of the chamber, the storage portion, the channel, and the opening is not particularly limited. In the present embodiment, preferably, the chamber, the storage portion, the channel, and the opening are arranged such that, when at least one of a centrifugal force and an inertial force is applied to the cartridge, the sample infused from the opening is transferred to the chamber through the channel while the first liquid reagent stored in the storage portion is transferred to the chamber. Hereinafter, an example of the base body constituting the cartridge will be described with reference to the drawings. However, the cartridge of the present embodiment is not limited to this example. With reference to FIG. 4B, a cartridge 200 is formed of a disk-shaped base body 200a. While in FIG. 4B the cartridge has a disk shape, the cartridge is not limited to this shape. The cartridge of the present embodiment is preferably a plate-like cartridge. The shape of the cartridge may be a disk shape or a rectangular shape, for example. The cartridge may have a protruding portion or the like.

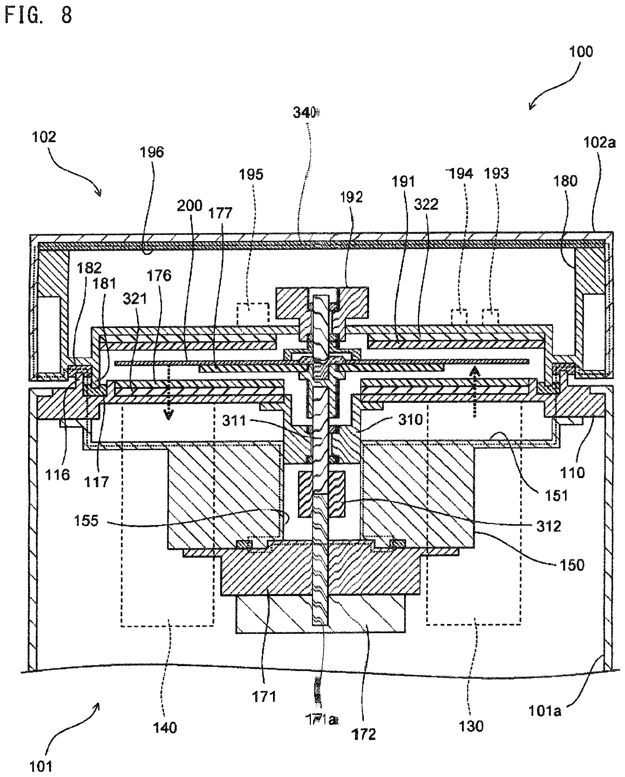

The base body 200a includes a hole 201, chambers 211 to 216, a channel 220, six storage portions 231, a storage portion 232, an opening 241, a separator 242, and a channel 243. The hole 201 penetrates the base body 200a at the center of the base body 200a. The cartridge 200 is placed on an analyzer 100 so that the center of the hole 201 is aligned with a rotation shaft 311 described later (refer to FIG. 8). Hereinafter, a radial direction and a circumferential direction, of a circle around the rotation shaft 311, are simply referred to as "radial direction" and "circumferential direction", respectively. The chambers 211 and 216 are arranged side by side in the circumferential direction, near the outer circumference of the base body 200a.

The chambers 211 to 216, the channel 220, the six storage portions 231, the storage portion 232, the separator 242, and the channel 243 are formed as recesses in the base body 200a. Further, the base body 200a includes an index which defines a reference position (home position) in the circumferential direction of the cartridge 200. The index is, for example, a hole provided in the base body 200a. A sensor (e.g., an optical sensor) opposing the index is disposed at a predetermined position in a body 101, and the sensor detects the index.

The chambers 211 to 216 are containers provided in the cartridge 200 to store a liquid containing a test substance therein. Each of the chambers does not necessarily have to store the liquid all the time, and only has to have a spatial expanse for storing the liquid therein. The channel 220 is a flow path provided in the cartridge 200 to transfer the sample together with the carrier particles from one chamber to another chamber. The channel 220 has an arc-shaped region 221 extending in the circumferential direction, and six regions 222 extending in the radial direction. The region 221 is connected to the six regions 222. The six regions 222 are connected to the chambers 211 to 216, respectively. The six storage portions 231 are connected to the channel 220 via flow paths, and are present on extensions of the regions 222 connected to the chambers 211 to 216, respectively. The storage portion 232 is connected, via a flow path, to a flow path that connects the region 222 connected to the chamber 216 with the storage portion 231 on an extension of the region 222 connected to the chamber 216.

Each storage portion 231 is provided with a seal 231a at an upper surface thereof on the inner side in the radial direction. The seal 231 a is opened when being pressed from above by a seal opening unit 195 described later. Thereby, the inside of the storage portion 231 is communicated with the outside of the cartridge 200 at the position of the seal 231 a. Likewise, the storage portion 232 is also provided with a seal 232a at an upper surface thereof on the inner side in the radial direction. The seal 232a is opened by being pressed from above by the seal opening unit 195. Thereby, the inside of the storage portion 232 is communicated with the outside of the cartridge 200 at the position of the seal 232a.

The seal 231 a and the seal 232a may also be formed at the upper surfaces of the storage portions 231 and 232, respectively, on the outer side in the radial direction, although these seals are not shown in FIG. 4B. By closing each storage portion with two seals, the liquid reagent or the like stored in the storage portion can be stably preserved until the cartridge is used. These seals are also opened by being pressed from above by the seal opening unit 195. Thus, the storage portions 231 and 232 are connected to the corresponding chambers via flow paths.

The sample containing the test substance is infused into the separator 242 via the opening 241. When the sample is blood, the blood can be separated into blood cells and plasma by a centrifugal force in the separator 242. The plasma separated through the separator 242 moves to the channel 243. A hole 243a is provided at an upper surface of the channel 243 on the inner side in the radial direction. The plasma, which is positioned in a region 243b in the channel 243, moves to the chamber 211 due to a centrifugal force when the cartridge 200 is rotated. Thus, a predetermined amount of plasma is transferred to the chamber 211.

In FIG. 4B, the components of the base body 200a are formed only in the one-third area of the base body 200a. However, the present embodiment is not limited to this example. The set of the components shown in FIG. 4B may be formed in the remaining two-third area. That is, three sets of the components may be provided in the base body 200a.

In the cartridge of the present embodiment, the carrier particles are immobilized to an inner wall of the chamber by means of a solid mixture containing sugar and protein. In this specification, "immobilizing" means fixing a target substance so as not to move from a predetermined position. The solid mixture containing sugar and protein (hereinafter also simply referred to as "solid mixture") is a medium for immobilizing the carrier particles to the inner wall of the chamber. The solid mixture itself is also adhered and immobilized to the inner wall of the chamber.

In the present embodiment, examples of the manner of immobilizing the carrier particles by means of the solid mixture include: covering the carrier particles with the solid mixture; and dispersing or enclosing the carrier particles in the solid mixture. Examples of the state in which the carrier particles do not move from a predetermined position include a state in which the carrier particles are always stationary at a predetermined position on the inner wall. However, the present embodiment is not limited to these examples. For example, when the carrier particles are dispersed or enclosed in a viscous or elastic solid mixture, the carrier particles may move with deformation of the solid mixture. In this case, as long as the solid mixture is immobilized to the predetermined position on the inner wall of the chamber, the carrier particles being dispersed or enclosed in such a solid mixture is included in the state in which the carrier particles do not move from a predetermined position.

In this specification, "carrier particles" and "solid mixture containing sugar and protein" are mentioned as different substances. That is, unless specifically mentioned, the term "solid mixture containing sugar and protein" itself does not intend to further contain "carrier particles". Further, in this specification, "carrier particles" and "solution containing sugar and protein" are mentioned as different substances. That is, unless specifically mentioned, the term "solution containing sugar and protein" itself does not intend to further contain "carrier particles".

The solid mixture is a composition generated by drying the solution containing sugar and protein. The solid mixture may be a hard solid obtained by completely drying and solidifying the solution described above, or may be a viscous or elastic solid (e.g., gel or semisolid), as long as the solid mixture does not move from the predetermined position on the inner wall of the chamber. However, when the solid mixture is a hard solid, a cracked solid mixture is excluded from the present embodiment. A cracked solid mixture may be separated from the chamber due to impact during transport, and become incapable of immobilizing the carrier particles.

In the present embodiment, the immobilized carrier particles may be or may not be in contact with the inner wall. For example, the carrier particles may be immobilized to the inner wall of the chamber such that the carrier particles in contact with the inner wall are covered with the solid mixture. Alternatively, the carrier particles may be immobilized to the inner wall of the chamber such that the carrier particles are dispersed or enclosed in the solid mixture adhered to the inner wall. In this case, even if the carrier particles are not in contact with the inner wall, the carrier particles are indirectly immobilized to the inner wall by means of the solid mixture. In the present embodiment, the solid mixture comes into contact with a liquid mixture containing the test substance, the first capture substance, and the first liquid reagent, and dissolves in the liquid mixture, whereby the carrier particles are separated from the inner wall and dispersed into the mixture.

The carrier particles are desirably not bound to the inner wall of the chamber to facilitate separation of the carrier particles from the inner wall after dissolution of the solid mixture. For example, it is preferable that there is no binding due to covalent bond, physical adsorption, or the like between the carrier particles and the inner wall of the chamber.

The position, on the inner wall of the chamber, at which the carrier particles are immobilized is not particularly limited, and may be a region of the inner wall or the entire surface of the inner wall. The present embodiment may include a case where part of the carrier particles is immobilized to a region 222 (refer to FIG. 4B) connected to a chamber. Since the carrier particles are immobilized, the carrier particles are prevented from separating from the chamber due to vibration, impact, or the like during transport of the cartridge as a product. The chamber to which the carrier particles are immobilized may be any of the plurality of chambers, but preferably is a chamber to which the sample infused from the opening is transferred first. For example, with reference to FIG. 4B, the carrier particles are preferably immobilized to the chamber 211.

The sugar contained in the solid mixture is not particularly limited as long as it is water-soluble and does not inhibit specific binding between the test substance and the capture substance. Preferably, the sugar is easily soluble in water. Such a sugar may be selected as appropriate from monosaccharides and disaccharides. Examples of the sugar may include sucrose, trehalose, glucose, lactose, fructose, maltose, and galactose. The sugar may include one, or two or more kinds of sugars. In the present embodiment, sucrose is particularly preferable.

The protein contained in the solid mixture is not particularly limited as long as it is water-soluble and does not inhibit specific binding between the test substance and the capture substance. A composition containing a protein may be used. In the present embodiment, a protein having a function of preventing aggregation of the carrier particles and/or deterioration of the capture substance is preferably used. Such a protein may be selected as appropriate from among proteins that are generally used as stabilizing agents or blocking agents in immunological measurement. Examples of the protein include albumin, crystallin, casein, normal serum protein, collagen, gelatin, Gelysate, skim milk, lactic acid ferment, and decomposition products thereof. The protein may include one, or two or more kinds of proteins. When an animal protein is used, the origin of the protein is not particularly limited. In the present embodiment, serum albumin is preferable, and bovine serum albumin (BSA) is particularly preferable.

In the cartridge of the present embodiment, immobilization of the carrier particles to the inner wall by means of the solid mixture is performed as follows, for example. The carrier particles are dispersed into the solution containing sugar and protein to prepare a suspension of the carrier particles. Then, this suspension is dried on the inner wall, whereby the carrier particles immobilized to the inner wall by the solid mixture is obtained. In the suspension of the carrier particles, the concentration of the protein is preferably not less than 0.1 mass% and not greater than 20 mass%, and more preferably not less than 1.0 mass% and not greater than 5 mass%. In the suspension of the carrier particles, the concentration of the sugar is preferably not less than 0.5 mass% and not greater than 30 mass%, and more preferably not less than 1.0 mass% and not greater than 15 mass%. In this specification, "mass%" may be replaced by "weight%".

In the solid mixture containing sugar and protein, the mass ratio of the sugar to the protein (mass of sugar/mass of protein) is generally not less than 0.1 and not greater than 100, preferably not less than 0.5 and not greater than 30, and more preferably not less than 1 and not greater than 10. In the cartridge of the present embodiment, the mass ratio of the carrier particles to the protein contained in the solid mixture (mass of carrier particles/mass of protein) is generally not less than 0.05 and not greater than 10, preferably not less than 0.1 and not greater than 7, and more preferably not less than 0.5 and not greater than 5. In the present embodiment, since the sample is processed in the cartridge, the liquid inside the cartridge cannot be agitated by direct means such as pipetting. However, by adjusting the mass ratios of the sugar and the carrier particles to the protein within the ranges described above, the solid mixture quickly dissolves in the liquid containing the test substance, which allows the carrier particles to separate from the inner wall of the chamber and disperse into the liquid.

Regarding the material of the carrier particles, the same material as particles generally used as a solid phase carrier in immunological measurement can be used. For example, metal particles, resin particles, or silica particles can be used. Examples of the metal particles include: particles of gold, silver, copper, iron, aluminum, manganese, nickel, and titanium; particles of oxides thereof; and particles of alloys thereof. Examples of the resin particles include polystyrene particles, and latex particles. In a preferable embodiment, the carrier particles are magnetized particles (hereinafter also referred to as "magnetic particles"). Examples of the magnetic particles include particles that are known in this technical field and contain, as bases, iron oxide (Fe₂O₃ and/or Fe₃O₄), chrome oxide, cobalt, ferrite, magnetite, and the like.

The size of the carrier particles is not particularly limited as long as the carrier particles can pass through the channel. For example, particles having a mean particle diameter not less than 10 nm and not greater than 100 µm can be used. The mean particle diameter of the carrier particles is a volume-based median diameter which is measured by a particle size distribution measuring device using a laser diffraction scattering method. Examples of the particle size distribution measuring device include "Microtrac MT3000II" manufactured by Nikkiso Co., Ltd. In this specification, "particle size" means "particle diameter". The shape of the carrier particles is not particularly limited. For example, the particle shape may be any of sphere, rectangular parallelepiped, cube, trigon, and similar shapes thereto.

The carrier particles each preferably have a surface to which the first capture substance can be immobilized. Such a surface can be determined according to a binding manner between the carrier particle and the first capture substance. For example, when the first capture substance is a protein, the surface of the carrier particle may be formed of a material that physically adsorbs the protein. When the first capture substance is an antibody, molecules that specifically bind to the antibody may be immobilized onto the surface of the carrier particle. Examples of the molecules that specifically bind to the antibody include protein A, and protein G. A combination of materials interposed between the first capture substance and the carrier particle may be used. Examples of such a combination of materials include a combination of biotin and avidin (or streptavidin), and a combination of hapten and anti-hapten antibody. Examples of the combination of hapten and anti-hapten antibody include a combination of dinitrophenyl

(DNP) group and anti-DNP antibody. For example, when the first capture substance is modified with biotin, avidin or streptavidin may be immobilized to the surface of the carrier particle.

In the cartridge of the present embodiment, the first liquid reagent is stored in the storage portion. The first liquid reagent is a medium for diluting the sample to an appropriate concentration, and causing the carrier particles immobilized to the inner wall of the chamber to be dispersed therein. Examples of the first liquid reagent include water, saline, phosphate buffer solution (PBS), and Good buffer solution. Examples of the Good buffer solution include MES, Bis-Tris, ADA, PIPES, Bis-Tris-Propane, ACES, MOPS, MOPSO, BES, TES, HEPES, HEPPS, Tricine, Tris, Bicine, and TAPS.

The first capture substance is not particularly limited as long as it is a substance that specifically binds to the test substance. Various manners of binding the capture substance and the test substance are conceivable according to the kind of the test substance. Examples of the manner of binding include: binding using antigen-antibody reaction; binding using formation of complementary strand of nucleic acid; and binding between a receptor and a ligand. Therefore, the first capture substance may be selected as appropriate according to the kind of the test substance (antibody, antigen, nucleic acid, receptor, ligand, aptamer, etc.). In the present embodiment, the test substance is preferably detected by using antigen-antibody reaction. Therefore, the first capture substance is preferably an antibody or an antigen that specifically binds to the test substance.

In this specification, "antibody" includes a monoclonal antibody, a polyclonal antibody, and antibody fragments such as Fab and F(ab')₂. In this specification, "nucleic acid" as the capture substance includes not only DNA and RNA but also artificial nucleic acids such as Peptide Nucleic Acid (PNA), Locked Nucleic Acid (LNA), and Bridged Nucleic Acid (BNA).

The first capture substance may be labeled with a labeling substance. As the labeling substance, a substance that generates a signal (hereinafter also referred to as "signal generating substance") or a substance that catalyzes reaction of another substance and causes the substance to generate a detectable signal, can be used. Examples of the signal generating substance include a fluorescent substance, and a radioisotope. The substance that catalyzes reaction of another substance and causes the substance to generate a detectable signal may be an enzyme, for example. Examples of the enzyme include peroxidase, alkaline phosphatase, β-galactosidase, glucose oxidase, tyrosinase, acid phosphatase, and luciferase. Examples of the fluorescent substance include: fluorescent dyes such as fluorescein isothiocyanate (FITC), rhodamine, Alexa Fluor (registered trademark), and a cyanine dye; and fluorescent proteins such as GFP. Examples of the radioisotope include ¹²⁵I, ¹⁴C, and ³²P. Among them, as the labeling substance, an enzyme is preferable, and peroxidase and alkaline phosphatase are particularly preferable.

In the cartridge of the present embodiment, a site where the first capture substance is stored is not particularly limited. For example, a liquid reagent containing the first capture substance may be stored in the storage portion. In order to reduce the number of reagents to be mounted on the cartridge, the first capture substance is preferably contained in the first liquid reagent, or immobilized to the surfaces of the carrier particles in advance. Immobilization of the first capture substance to the carrier particles may be performed by bringing the first capture substance described above into contact with particles having surfaces to which the first capture substance can be immobilized. Alternatively, the first capture substance may be bound, through covalent bond, to the surfaces of the carrier particles by using a known cross-linking agent.

In the present embodiment, the cartridge may be further provided with a second liquid reagent containing a second capture substance that specifically binds to the test substance. In this case, the second liquid reagent is preferably stored in a storage portion different from the storage portion where the first liquid reagent is stored. For example, with reference to FIG. 4B, when the first liquid reagent is stored in the storage portion 231 positioned in the radial direction of the chamber 211, the second liquid reagent may be stored in the storage portion 231 positioned in the radial direction of the chamber 212.

The second capture substance is not particularly limited as long as it is a substance that specifically binds to the test substance. The kind and the like of the second capture substance are the same as described for the first capture substance. The first capture substance and the second capture substance may be substances of the same kind, or may be substances of different kinds. As an example of a case where the first capture substance and the second capture substance are of the same kind, there is a case where both the capture substances are antibodies. As an example of a case where the first capture substance and the second capture substance are of different kinds, there is a case where the first capture substance is an aptamer and the second capture substance is an antibody. When the first capture substance is not labeled with a labeling substance, the second capture substance is preferably labeled with a labeling substance.

A solvent for the second liquid reagent is not particularly limited as long as it can dissolve the second capture substance. Examples of the solvent include water, saline, phosphate buffer solution (PBS), and Good buffer solution.

In order to avoid competition between the first capture substance and the second capture substance, the first capture substance and the second capture substance preferably bind to different positions on the test substance. For example, when the first and second capture substances are antibodies and the test substance is an antigen, an epitope of the test substance to which the first capture substance binds is preferably different from an epitope of the test substance to which the second capture substance binds. When the first and second capture substances and the test substance are nucleic acids, a base sequence of the test substance to which the first capture substance binds is preferably different from a base sequence of the test substance to which the second capture substance binds. In this embodiment, since one test substance is captured by two kinds of capture substances, specificity in detection is improved.

In the present embodiment, the cartridge may be further provided with a washing liquid for removing unreacted free ingredients. The composition of the washing liquid is not particularly limited as long as a complex formed on the carrier particles is not impaired by the washing liquid. In the present embodiment, the washing liquid may be a washing liquid that is generally used in immunological measurement. In the cartridge of the present embodiment, the washing liquid is preferably stored in a storage portion different from the storage portions where the first and second liquid reagents are respectively stored. For example, with reference to FIG. 4B, when the first liquid reagent is stored in the storage portion 231 positioned in the radial direction of the chamber 211 and the second liquid reagent is stored in the storage portion 231 positioned in the radial direction of the chamber 212, the washing liquid may be stored in the storage portion 231 positioned in the radial direction of the chamber 213. The washing liquid may include one, or two or more kinds of washing liquids. For example, when the cartridge is provided with three kinds of washing liquids, with reference to FIG. 4B, the washing liquids may be stored in three storage portions 231 positioned in the radial directions of the chambers 213 to 215, respectively.

When an enzyme is used as the labeling substance described above, the cartridge may be further provided with a substrate solution of the enzyme. In the cartridge of the present embodiment, the substrate solution is preferably stored in a storage portion capable of supplying the content thereof to a chamber to which the carrier particles are finally transferred. For example, with reference to FIG. 4B, the substrate solution is preferably stored in the storage portion 231 positioned in the radial direction of the chamber 216, or the storage portion 232.

The cartridge may be further provided with a buffer (hereinafter also referred to as "reaction buffer") suitable for reaction between an enzyme and a substrate, according to need. The reaction buffer is selected as appropriate according to the kinds of the enzyme and the substrate. With reference to FIG. 4B, the reaction buffer is preferably stored in the storage portion 231 positioned in the radial direction of the chamber 216. In this case, the substrate solution is preferably stored in the storage portion 232.

The substrate can be selected as appropriate from among substrates known in the technical field, according to the enzyme. When the enzyme is alkaline phosphatase, examples of the substrate include: chemiluminescent substrates such as CDP-Star (registered trademark) (disodium 4-chloro-3-(methoxyspiro[1,2-dioxetane-3,2'-(5'-chloro)tricyclo[3.3.1.1^{3,7}]decan]-4-yl)phenylphosphate), and CSPD (registered trademark) (disodium 3-(4-methoxyspiro[1,2-dioxetane-3,2-(5'-chloro)tricyclo[3.3.1.1^{3,7}]decane]-4-yl)phenylphosphate); and chromogenic substrates such as 5-bromo-4-chloro-3-indolyl phosphate (BCIP), disodium 5-bromo-6-chloroindolyl phosphate, and p-nitrophenyl phosphate. When the enzyme is peroxidase, examples of the substrate include: chemiluminescent substrates such as luminol and derivatives thereof; and chromogenic substrates such as 2,2'-azinobis(3-ethylbenzothiazoline-6-sulphonic acid ammonium salt) (ABTS), 1, 2-phenylenediamine (OPD), and 3,3',5,5'-tetramethylbenzidine (TMB).

### [2. Method for producing cartridge for sample analysis]

In a method for producing the cartridge of the present embodiment (hereinafter also simply referred to as "production method"), first, the carrier particles are dispersed into the solution containing sugar and protein to prepare a suspension of the carrier particles. When this suspension is dried on a solid phase, the carrier particles immobilized onto the solid phase by the solid mixture are obtained. The kinds of the sugar, the protein, and the carrier particles contained in the suspension are the same as described above. A solvent for the solution containing sugar and protein is not particularly limited as long as it can dissolve the sugar and the protein. Examples of the solvent include water, saline, and Good buffer solution.

If the concentrations of the sugar and the protein in the suspension of the carrier particles are too low, the dried and immobilized solid mixture is hard to dissolve, which makes dispersion of the carrier particles difficult. Therefore, in the suspension of the carrier particles, the concentration of the protein is preferably not less than 0.1 mass%, and more preferably not less than 1.0 mass%. The concentration of the sugar is preferably not less than 0.5 mass%, and more preferably not less than 1.0 mass%. If the concentration of the sugar in the suspension of the carrier particles is too high, the suspension is hard to be dried. Therefore, the concentration of the sugar in the suspension of the carrier particles is preferably not greater than 30 mass%, and more preferably not greater than 15 mass%. The upper limit of the concentration of the protein in the suspension of the carrier particles is not particularly limited, but is generally not greater than 20 mass%, and preferably not greater than 5 mass%.

In the suspension of the carrier particles, the mass ratio of the sugar to the protein (mass of sugar/mass of protein) is generally not less than 0.1 and not greater than 100, preferably not less than 0.5 and not greater than 30, and more preferably not less than 1 and not greater than 10. In the suspension of the carrier particles, the mass ratio of the carrier particles to the protein (mass of carrier particles/mass of protein) is generally not less than 0.05 and not greater than 10, preferably not less than 0.1 and not greater than 5, and more preferably not less than 0.5 and not greater than 7. By adjusting the mass ratios of the sugar and the carrier particles to the protein within the ranges described above, the solid mixture easily dissolves in the liquid containing the test substance, which allows the carrier particles to quickly separate from the inner wall of the chamber and disperse into the liquid.

Next, on the substrate in which the chamber for storing therein the liquid containing the test substance is formed, the suspension described above is supplied to a region where the chamber is formed. The details of the substrate are the same as those described for the cartridge of the present embodiment. For example, when a plurality of recesses to be chambers are formed in the substrate, the suspension is supplied to at least one of the recesses to be the chambers. Supply of the suspension is performed by placing the suspension on the region on the substrate. The amount of the suspension to be supplied is not particularly limited, and may be not less than 1% and not greater than 75% of the volume of the chambers, for example.

The chamber to which the carrier particles are finally immobilized may be any of the plurality of chambers, but is preferably a chamber to which the sample infused from the opening is transferred first. For example, with reference to FIG. 4B, the suspension is preferably supplied to the region, on the substrate, where the chamber 211 is to be formed.

Then, the supplied suspension is dried to immobilize the carrier particles onto the substrate. The drying method is not particularly limited, and air drying, heat drying, or freeze drying can be used, for example. A desiccant, a desiccator, a vacuum dryer, and the like may be used according to need. In view of operational convenient, the cartridge base body to which the suspension is supplied is preferably subjected to air drying under normal temperature and normal pressure. The drying time is not particularly limited, and is preferably not less than 1 hour and not more than 30 hours in the case of air drying.

After the drying, a chamber for enclosing the immobilized carrier particles is formed. When a plurality of recesses to be chambers are formed in the substrate, other chambers that do not enclose the carrier particles may be further formed. In the present embodiment, it is preferable to form storage portions. When a plurality of chambers are formed, it is preferable to form a channel for connecting the chambers. For example, when recesses to be chambers, storage portions, and a channel are formed in the substrate, a film that covers the entire surface of the substrate may be bonded to the substrate. Alternatively, to this substrate, another substrate in which recesses to be chambers, storage portions, and a channel are formed in a symmetrical manner with respect to those of the substrate may be bonded. Thus, a cartridge for sample analysis in which the carrier particles are immobilized onto the inner wall of a chamber is obtained.

In the present embodiment, in the respective storage portions formed as described above, the first liquid reagent, the second liquid reagent, the washing liquid, the substrate solution, and the reaction buffer are preferably stored. Details of these reagents and the like are the same as those described for the cartridge for sample analysis of the present embodiment.

### [3. Method for detecting test substance using cartridge for sample analysis]

In a test substance detection method (hereinafter also simply referred to as "detection method") of the present embodiment, a cartridge for sample analysis provided with carrier particles is used. In the cartridge for sample analysis, the carrier particles are immobilized to the inner wall of a chamber formed in the cartridge, by means of a solid mixture containing sugar and protein. Details, such as the composition and mass ratio, of the solid mixture containing sugar and protein are the same as those described for the cartridge of the present embodiment. In the detection method of the present embodiment, it is particularly preferable to use the cartridge of the present embodiment described above.

In the detection method of the present embodiment, first, the carrier particles immobilized to the inner wall of the chamber formed in the cartridge for sample analysis, a sample containing a test substance, a first capture substance that specifically binds to the test substance, and a first liquid reagent are brought into contact with each other in the chamber. Details of the sample containing the test substance, the carrier particles, the first capture substance, and the first liquid reagent are the same as those described for the cartridge of the present embodiment.

When the sample is infused into the cartridge from the opening and at least one of a centrifugal force and an inertial force is applied to the cartridge, the sample is transferred through a flow path to the chamber to which the carrier particles are immobilized. At this time, by the applied centrifugal force and/or inertial force, the first liquid reagent stored in a storage portion is also transferred to the chamber to which the carrier particles are immobilized. The first capture substance is preferably contained in the first liquid reagent, or immobilized to the surfaces of the carrier particles in advance. Thus, the carrier particles, the sample containing the test substance, the first capture substance, and the first liquid reagent can be brought into contact with each other in the chamber. When the first liquid reagent containing the first capture substance is used, the carrier particles are preferably particles having surfaces to which the first capture substance can be immobilized. Details of such surfaces are the same as those described for the cartridge of the present embodiment.

In the present embodiment, since the sample infused from the opening is transferred through the flow path to the chamber to which the carrier particles are immobilized, this sample is preferably liquid. When the sample is not liquid, the sample is preferably liquified through pretreatment. The liquid sample is not limited to a solution, and may be a suspension, a sol, or the like. As a method of the pretreatment, a known method can be selected according to the kind of the test substance. For example, when the sample is a solid tissue extracted from an organism, the solid tissue is homogenized in a buffer solution containing a surfactant, and a homogenate is separated and removed by centrifugation or the like, whereby the solid tissue can be liquified. In this case, supernatant after the centrifugation can be infused into the cartridge.

Next, in the present embodiment, the carrier particles are dispersed by agitation into a liquid mixture containing the first capture substance and the first liquid reagent. By the contact described above, the solid mixture containing sugar and protein starts to dissolve in the liquid mixture. By agitating the cartridge, the solid mixture can be completely dissolved. Thereby, the carrier particles can be caused to separate from the inner wall of the chamber and disperse into the liquid mixture. The agitation is preferably performed such that at least one of a centrifugal force and an inertial force is applied to the cartridge, and the magnitudes of the centrifugal force and/or the inertial force are changed with time. For example, when the cartridge is a disk-shaped cartridge as shown in FIG. 4B, a centrifugal force can be applied to the cartridge by rotating the cartridge, and the liquid inside the cartridge can be agitated by rapidly changing the rotation speed. An example of the agitation is to repeat, for 30 seconds, acceleration of the rotation speed from 50 rpm to 250 rpm within 0.02 second and deceleration of the rotation speed from 250 rpm to 50 rpm within 0.02 second.

After the agitation, in the present embodiment, a complex containing the test substance and the first capture substance is formed on the dispersed carrier particles. When the carrier particles are particles to which the first capture substance is immobilized, the first capture substance on the carrier particles specifically binds to the test substance, whereby a complex can be formed on the carrier particles. When the first liquid reagent contains the first capture substance, a complex can be formed on the carrier particles by using particles having surfaces to which the first capture substance can be immobilized.

The temperature and the reaction time when the complex is formed are not particularly limited. For example, incubation may be performed at 37 to 42°C for 60 to 600 seconds.

In the present embodiment, an operation to remove free ingredients which are not contained in the complex may be performed. This removal of the free ingredients is performed by separating molecules (Bounds) immobilized onto the carrier particles from molecules (Frees) which are not immobilized to the carrier particles and are in their free states. This separation is also called B/F separation. Examples of the free ingredients not contained in the complex include: the unreacted capture substance; and the test substance that does not bind to the capture substance. When the carrier particles are, for example, magnetic particles, B/F separation can be performed by transferring the magnetic particles to another chamber in the cartridge by means of a magnet or a magnetic collection device, and supplying a washing liquid to the chamber.

In the present embodiment, the test substance contained in the complex formed on the carrier particles is detected. In this specification, "detection" includes qualitative detection, quantitative detection, and semi-quantitative detection. The "semi-quantitative detection" means stepwise presentation of the content (or concentration) of the test substance in the sample, such as "negative", "weakly positive", "positive", and "strongly positive".

In the present embodiment, the test substance is preferably detected by bringing a labeling substance into indirect contact with the test substance contained in the complex, and detecting a signal based on the labeling substance. For example, when the first capture substance has been labeled with a labeling substance in advance, a signal based on the labeling substance is detected, and the test substance is detected on the basis of the signal. Details of the labeling substance are the same as those described for the cartridge of the present embodiment.

The method itself for detecting a signal based on a labeling substance is known in the technical field. A method for detecting a signal can be selected as appropriate according to the kind of the labeling substance. For example, when the labeling substance is an enzyme, a signal of light, color, or the like, which is generated by reaction between the enzyme and a substrate for the enzyme, is measured by using a known measurement device. Examples of the measurement device include a spectrophotometer, and a luminometer. When the labeling substance is a fluorescent substance, fluorescence as a signal can be measured by using a known device such as a fluorescence microplate reader. An excitation wavelength and a fluorescence wavelength can be determined as appropriate according to the kind of the used fluorescent substance.

In the present embodiment, a second liquid reagent containing a second capture substance that specifically binds to the test substance may be further added between the complex formation process and the complex detection process. Thereby, a complex containing the test substance, the first capture substance, and the second capture substance can be formed on the dispersed carrier particles. In this complex, the test substance is sandwiched between the first capture substance and the second capture substance. When the first capture substance is not labeled with a labeling substance, the second capture substance is preferably labeled with a labeling substance.

When the carrier particles are, for example, magnetic particles, addition of the second liquid reagent can be performed by transferring the magnetic particles to another cartridge by means of a magnet or a magnetic collection device, and supplying the second liquid reagent to the chamber. Details of the second capture substance and the second liquid reagent are the same as those described for the cartridge of the present embodiment.

In the present embodiment, the second capture substance is preferably a labeled antibody or a labeled antigen that specifically binds to the test substance. In this case, the test substance can be detected on the basis of the label of the labeled antibody or the labeled antigen contained in the complex.

The detection method of the present embodiment is preferably executed by using, for example, an analyzer dedicated for the cartridge for sample analysis, as shown in FIG. 4A. Hereinafter, an example of the detection method using the analyzer and the cartridge for sample analysis in which magnetic particles are dried and immobilized will be described with reference to FIG. 4A. However, the present embodiment is not limited to this example.

### <Example of structure of analyzer>

The analyzer 100 shown in FIG. 4A is a sample analyzer which detects a test substance in a sample by using an antigen-antibody reaction, and analyzes the test substance on the basis of the result of the detection. The analyzer 100 includes a body 101 and a lid 102. The body 101 supports the lid 102 so that the lid 102 is openable/closable with respect to the body 101. An upper portion of the body 101 is covered with a mounting member 110, and a cartridge 200 is placed in a center portion of an upper surface of the mounting member 110. The analyzer 100 sequentially transfers magnetic particles stored in the cartridge 200 into a plurality of chambers formed in the cartridge 200 to cause the magnetic particles to carry a complex composed of a test substance and a capture substance labeled with a labeling substance, and detects the test substance on the basis of the labeling substance.

In the body 101, the mounting member 110 is disposed on an upper surface of the body 101 opposed to the lid 102. The body 101, except the upper surface thereof, is covered with a casing 101 a. In the lid 102, a mounting member 180 is disposed on a lower surface of the lid 102 opposed to the body 101. The lid 102, except the lower surface thereof, is covered with a casing 102a. When the cartridge 200 is mounted/demounted, the lid 102 is opened as shown in FIG. 4A.

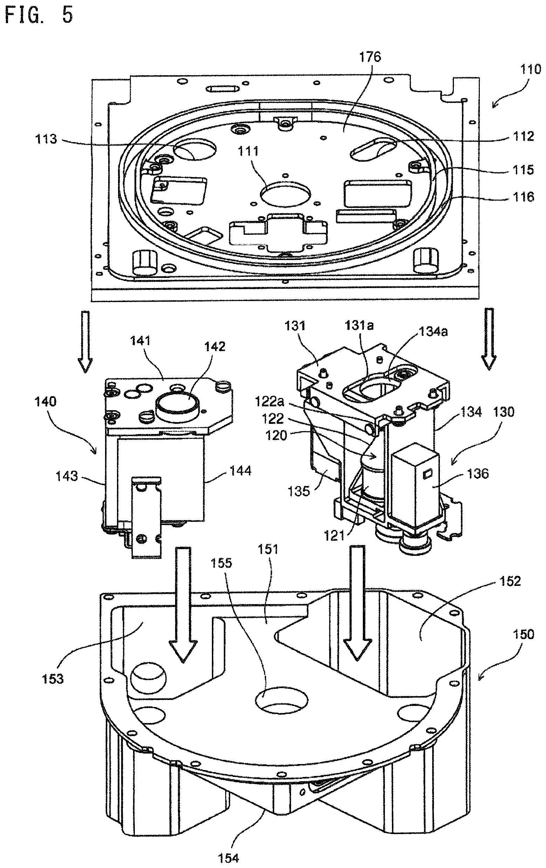

FIG. 5 shows the structure of the mounting member 110 on the body 101 side, and the structures of a magnet, a movement mechanism, a detection unit, and a housing which are housed in the casing 101a in the analyzer 100, as viewed from diagonally above. As shown in FIG. 5, the mounting member 110 has a plurality of holes formed therein. In a hole 111, a rotation shaft 311 described later (refer to FIG. 8) is positioned. A hole 112 has an elongated shape in the radial direction. A movement mechanism 130 is disposed on a lower surface of the mounting member 110 via an attachment member 131. A detection unit 140 is disposed beneath a hole 113 of the mounting member 110 via an attachment member 141. Beneath other holes, an index sensor for detecting an index of the cartridge 200 is disposed. A controller 301 described later reads a detection signal from the index sensor, and controls a motor 171 described later to control the position of the cartridge 200 in the circumferential direction.

A housing 150 has a plurality of holes formed in an upper surface 151 thereof. A hole 155 allows the rotation shaft 311 described later (refer to FIG. 8) to pass therethrough. Housing portions 152 and 153 are formed as recesses denting downward from the upper surface 151. When the mounting member 110 is mounted on the housing 150, an outer periphery lower surface of the mounting member 110 is joined to an outer periphery upper surface of the housing 150. When the mounting member 110 is mounted on the housing 150, the movement mechanism 130 is housed in the housing portion 152 and the detection unit 140 is housed in the housing portion 153. The mounting member 110 and the housing 150 are made of a light-shielding resin, and the mounting member 110 and the housing 150 are black-colored in order to enhance the light-shielding effect.

The movement mechanism 130 causes a magnet 120 to move independently in the radial direction and in an up-down direction. The magnet 120 is movable in the radial direction in accordance with driving of a motor 135, and the magnet 120 is movable in the up-down direction in accordance with driving of a motor 136. The movement mechanism 130 includes a sensor (not shown) for detecting a reference position (home position) of the magnet 120 in the radial direction. The movement mechanism 130 also includes a home position sensor (not shown) for detecting a home position of the magnet 120 in the up-down direction. The controller 301 described later reads detection signals from the home position sensors, and controls the motors 135 and 136 to control the positions of the magnet 120 in the radial direction and the up-down direction.

When the magnet 120 is moved upward, an upper end of the magnet 120 protrudes upward from the hole 112 of the mounting member 110 through a hole 131a of the attachment member 131 and a hole 134a of a support portion 134, and approaches the cartridge 200. When the magnet 120 is moved downward, the upper end of the magnet 120 moves away from the cartridge 200.

The magnet 120 includes a permanent magnet 121 having a cylindrical shape and a magnetic substance 122 having a conical shape. The magnetic substance 122 is joined to an upper surface of the permanent magnet 121. A cylindrical tip portion 122a having a smaller diameter than the permanent magnet 121 is formed at an upper end of the magnetic substance 122.

The width of an edge of the magnet 120 on the cartridge 200 side, that is, the width of the tip portion 122a, is smaller than at least the minimum width of each region in the channel 220. Thereby, the complex collected by the magnet 120 can be smoothly moved in the channel 220 without being caught in the channel 220.

The detection unit 140 includes: a ring-shaped reflection member 142 fitted in a hole formed in the attachment member 141; a support portion 143; and a light detection unit 144. The light detection unit 144, at an inner side with respect to the reflection member 142, includes: a photodetector 144a (not shown in FIG. 5) for detecting light from above; and a light adjustment unit 160 (not shown in FIG. 5) for inserting/removing an ND filter in/from an optical path of light that travels toward the photodetector 144a from above. The photodetector 144a is implemented by, for example, a photo multiplier tube, a phototube, a photodiode, or the like.

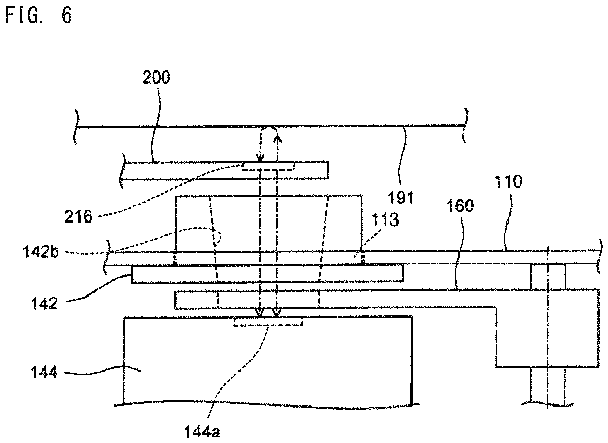

As shown in FIG. 6, light generated in the chamber 216 of the cartridge 200 spreads upward and downward from the cartridge 200. The light spreading downward from the cartridge 200 passes through a hole 142b of the reflection member 142, passes through the light adjustment unit 160, and is received by the photodetector 144a. The light spreading upward from the cartridge 200 is reflected by a plate member 191 described later, which is exposed at the lower surface of the lid 102, and returned to the chamber 216, and then is similarly received by the photodetector 144a. The light spreading upward from the cartridge 200 may be reflected by a mirror disposed on the plate member 191 of the lid 102.

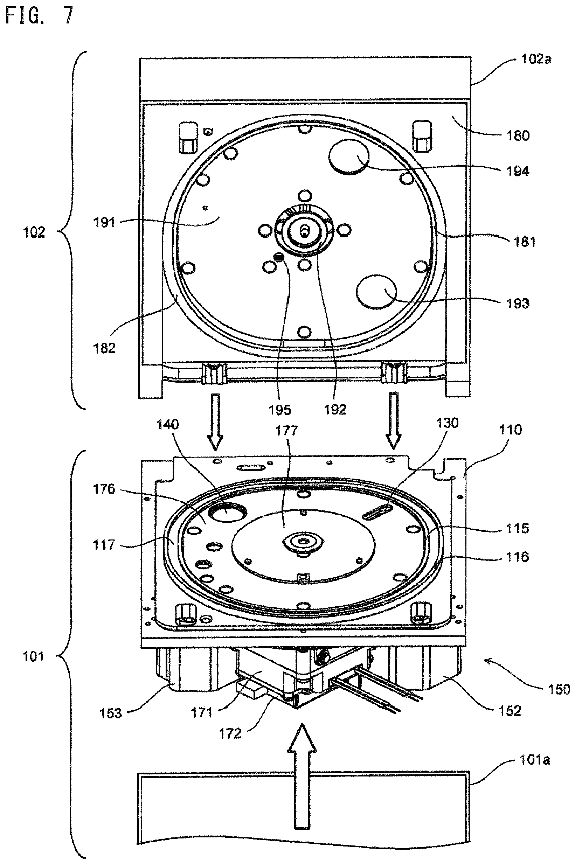

As shown in FIG. 7, a support member 177 is disposed at the center of the mounting member 110. The support member 177 is implemented as a turntable that supports and rotates the cartridge 200 mounted thereon. A protruding portion 115 and a protruding portion 116 are coaxially formed around the center portion where the support member 177 is disposed, and an elastic member 117 made of, for example, a light-shielding polyurethane resin, is disposed therebetween. The elastic member 117 is black-colored in order to enhance the light-shielding effect. The elastic member 117 is formed in a closed-loop shape. An upper surface of the elastic member 117 is an elastically deformable junction surface.

Each of the protruding portion 115 and the protruding portion 116 has an inner diameter larger than the outer diameter of the cartridge 200, so that the cartridge 200 being placed on the support member 177 falls inside the inner-side protruding portion 115. A plate member 176 is disposed on the upper surface of the mounting member 110 and within the protruding portion 115. The plate member 176 is made of a metal having high thermal conductivity. A heater 321 (not shown in FIG. 5) is disposed between a lower surface of the plate member 176 and the upper surface of the mounting member 110 (refer to FIG. 8). The housing 150 is combined with the lower surface side of the mounting member 110, and the combined housing 150 and mounting member 110 are housed in the casing 101 a to complete the body 101.

FIG. 7 also shows the lid 102 as viewed from the lower side thereof. The lid 102 includes: the mounting member 180; the plate member 191 disposed on the lower surface of the center portion of the mounting member 180; a clamper 192; an image capturing unit 193; a lighting unit 194; and a seal opening unit 195.

The mounting member 180 is made of a light-shielding resin, and is black-colored in order to enhance the light-shielding effect. The plate member 191 and the clamper 192 are disposed within the protruding portion 181 of the mounting member 180. The plate member 191 is made of a metal having high thermal conductivity, like the plate member 176. A heater 322 (not shown in FIG. 7) for heating the cartridge 200 to a temperature within a predetermined range is disposed between an upper surface of the plate member 191 and the lower surface of the mounting member 180 disposed above the plate member 191 (refer to FIG. 8). In overlapping portions of the lower surface of the mounting member 180, the plate member 191, and the heater 322, holes penetrating therethrough are provided at positions corresponding to the image capturing unit 193, the lighting unit 194, and the seal opening unit 195. Through these holes, the image capturing unit 193, the lighting unit 194, and the seal opening unit 195 directly oppose the upper surface of the cartridge 200. The image capturing unit 193, the lighting unit 194, and the seal opening unit 195 are disposed on an upper surface of the mounting member 180 (refer to FIG. 8).

The image capturing unit 193 captures an image of the cartridge 200 present in a dark space 340 so that the chambers 211 to 216 of the cartridge 200 can be visually observed. The image capturing unit 193 is implemented by, for example, miniature camera using a CCD image sensor, a CMOS image sensor, or the like. The lighting unit 194 irradiates the cartridge 200 with light when image capturing is performed by the image capturing unit 193. The lighting unit 194 is implemented by, for example, a light-emitting diode. The seal opening unit 195 includes: a member for pressing the seals 231a and 232a of the cartridge 200 from above; and a mechanism for driving this member.

The clamper 192 is disposed at the center of the mounting member 180. The closed-loop-shaped protruding portion 181 is formed on the lower surface of the mounting member 180. The protruding portion 181 protrudes downward along the circumferential direction. On the lower surface of the mounting member 180, a recess is formed outside the protruding portion 181, and an elastic member 182 is disposed in this recess. The elastic member 182 is made of, for example, a light-shielding polyurethane resin, and is black-colored in order to enhance the light-shielding effect. The elastic member 182 is formed in a closed-loop shape. A lower surface of the elastic member 182 is an elastically deformable junction surface.

FIG. 8 shows a state where the cartridge 200 is disposed on the analyzer 100, and the lid 102 is closed. As described above, the detection unit 140, and the movement mechanism 130 holding the magnet 120 are disposed on the lower surface of the mounting member 110, and the image capturing unit 193, the lighting unit 194, and the seal opening unit 195 are disposed on the upper surface of the mounting member 180. In FIG. 8, positions where these components are disposed are represented by broken lines.

As shown in FIG. 8, a mounting member 310 rotatably supports the rotation shaft 311 extending in the up-down direction. The rotation shaft 311, in the hole 155, is fixed to a drive shaft 171 a of the motor 171 by means of a fixing member 312. The motor 171 is implemented by a stepping motor. The drive shaft 171 a of the motor 171 extends to the inside of the hole 155. The mounting member 310 is disposed in an upper portion of the hole 155.

The support member 177 for supporting the lower surface of the cartridge 200 is fixed to an upper portion of the rotation shaft 311 via a predetermined member. When the motor 171 is driven to rotate the drive shaft 171 a, a rotation driving force is transmitted to the support member 177 via the rotation shaft 311. Thereby, the cartridge 200 mounted on the support member 177 rotates around the rotation shaft 311 and the drive shaft 171 a. When the cartridge 200 is placed on the support member 177 and the lid 102 is closed, the clamper 192 presses an inner circumferential portion of the upper surface of the cartridge 200 so that the cartridge 200 is rotatable.

When lid 102 is closed, the protruding portion 116 of the mounting member 110 is pressed and stuck onto the lower surface of the elastic member 182 of the mounting member 180. The protruding portion 181 of the mounting member 180 is pressed and stuck onto the upper surface of the elastic member 117 of the mounting member 110. Thus, the dark space 340 represented by a dotted line in FIG. 8 is formed.

The analyzer 100 further includes, inside the casing 101a, a control board on which a circuit functioning as the controller 301 is mounted. The controller 301 includes a CPU or an MPU, and a storage unit. The storage unit is implemented by, for example, a flash memory, a hard disk, or the like. The CPU or MPU described above executes processing based on a program stored in the storage unit in advance, and controls the operations of the respective components of the analyzer 100 to cause the analyzer 100 to perform measurement and analysis. That is, the controller 301 receives signals from the respective components of the analyzer 100, and controls the operations of the respective components of the analyzer 100. Thus, the function of the controller 301 is implemented by cooperation of software and hardware.

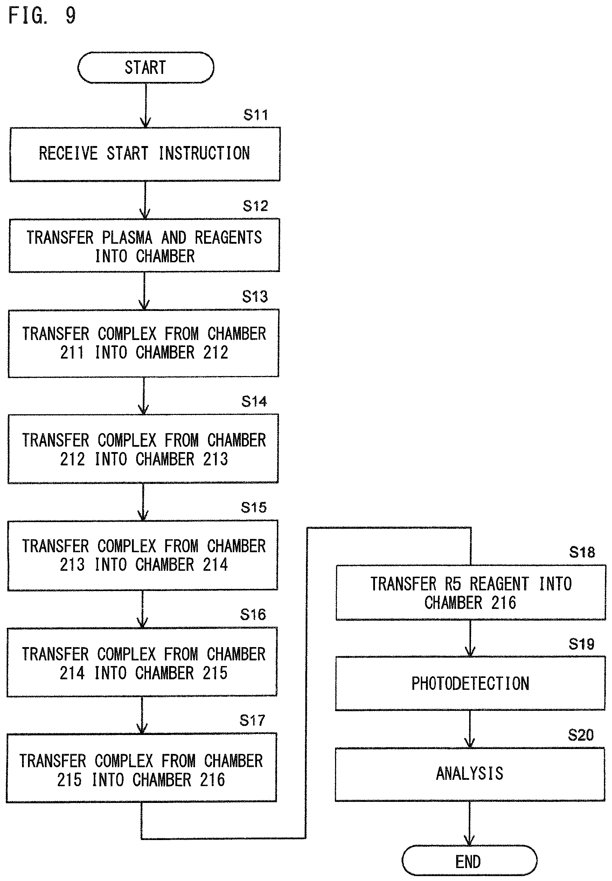

Next, an example of operation of the analyzer 100 will be described with reference to FIG. 9. First, an operator infuses, as a sample, blood collected from a subject into the cartridge 200 through the opening 241, and sets the cartridge 200 on the support member 177. In this example, a test substance is a surface antigen of hepatitis B virus in the blood (HBsAg). In this example, the blood infused into the cartridge 200 is separated into plasma and blood cells in the separator 242.

Predetermined liquid reagents are stored in the storage portions 231 and 232 and the chamber 211 of the cartridge 200 in advance. Specifically, an R1 reagent is stored in the storage portion 231 positioned in the radial direction of the chamber 211. In the chamber 211, magnetic particles dried and immobilized to the inner wall of the chamber 211 by means of a solid mixture containing sugar and protein (hereinafter also referred to as "R2 reagent") are stored. An R3 reagent is stored in the storage portion 231 positioned in the radial direction of the chamber 212. A washing liquid is stored in each of the storage portions 231 positioned in the radial direction of the chambers 213 to 215. An R4 reagent is stored in the storage portion 231 positioned in the radial direction of the chamber 216. An R5 reagent is stored in the storage portion 232.

The respective reagents used in this example will be described below. The R1 reagent is a buffer solution containing a biotin-bound anti-HBsAg monoclonal antibody as the first capture substance (capture antibody). The magnetic particles in the R2 reagent are streptavidin-bound magnetic particles. On the surfaces of the magnetic particles, streptavidin is immobilized. The R3 reagent is a buffer solution containing an alkaline phosphatase (ALP) labeled anti-HBsAg monoclonal antibody as the second capture substance (labeled antibody). The R4 reagent is a reaction buffer. The R5 reagent is a solution of CDP-Star (registered trademark) which is a chemiluminescent substrate of ALP.

In the control described below, the controller 301 obtains a rotation position of the drive shaft 171 a of the motor 171 on the basis of an output signal from an encoder 172 connected to the motor 171.

In step S11, the controller 301 receives a start instruction made by the operator via an input unit (not shown), and causes processes in step S12 and subsequent steps to be started. The input unit is a button, a touch panel, or the like which receives an operation performed by the operator. The input unit is provided on, for example, a side surface portion of the body 101 or an upper surface portion of the lid 102.

In step S12, the controller 301 executes a process for transferring the plasma and the reagents to the chambers. Specifically, the controller 301 drives the motor 171 to move the cartridge 200 in the circumferential direction, and drives the seal opening unit 195 to sequentially press down each of six seals 231a located at the position opposed to the seal opening unit 195. Then, the controller 301 drives the motor 171 to rotate the cartridge 200, thereby transferring, by a centrifugal force, the plasma positioned in the region 243b to the chamber 211, and transferring the reagents and the washing liquid stored in the six storage portions 231 to the chambers 211 to 216. Thus, the plasma and the R1 reagent flow into the chamber 211, and the magnetic particles dried and immobilized onto the inner wall of the chamber 211 are dispersed and mixed into the liquid flowed into the chamber 211. The R3 reagent is transferred into the chamber 212, the washing liquid is transferred into each of the chambers 213 to 315, and the R4 reagent is transferred into the chamber 216.

Further, in step S12, when transfer of the plasma and the reagents is finished, the controller 301 performs an agitation process for a predetermined period. Specifically, the controller 301 drives the motor 171 to switch the rotation speed of the motor 171 between two different rotation speeds at predetermined time intervals. Thereby, an Euler force generated in the circumferential direction is changed at the predetermined time intervals, and the liquid in each of the chambers 211 to 216 is agitated. This agitation process is performed not only in step S12 but also in steps S 13 to S 18 in a similar manner after the transfer process.

In step S12, when the plasma, the R1 reagent, and the magnetic particles are brought into contact with each other and subjected to the agitation process, the HBsAg in the plasma and the biotin-bound anti-HBsAg monoclonal antibody in the R1 reagent bind each other by antigen-antibody reaction, thereby forming a complex. In addition, through binding between the biotin bound to the antibody and the streptavidin at the surfaces of the magnetic particles, the antibody is immobilized onto the dispersed magnetic particles. Therefore, a complex containing the HBsAg and the biotin-bound anti-HBsAg monoclonal antibody is formed on the magnetic particles. Hereinafter, magnetic particles to which a complex containing a test substance and a capture substance is immobilized are also referred to as "complex".

Next, in step S13, the controller 301 causes the complex in the chamber 211 to be transferred from the chamber 211 to the chamber 212. Thereby, the complex formed in the chamber 211 and the R3 reagent are mixed together in the chamber 212. Then, when the agitation process is performed in step S13, the complex formed in the chamber 211 reacts with the labeled antibody contained in the R3 reagent. Thereby, a complex containing the test substance, the first capture substance (capture antibody), and the second capture substance (labeled antibody) is formed on the magnetic particles.

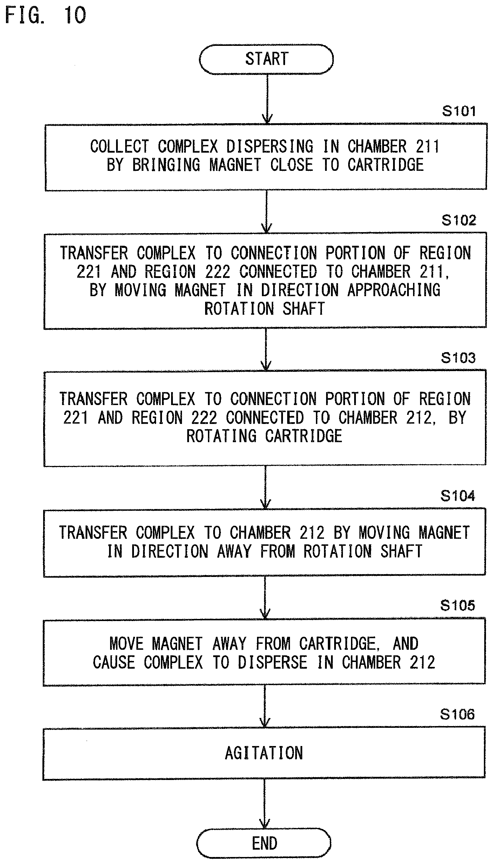

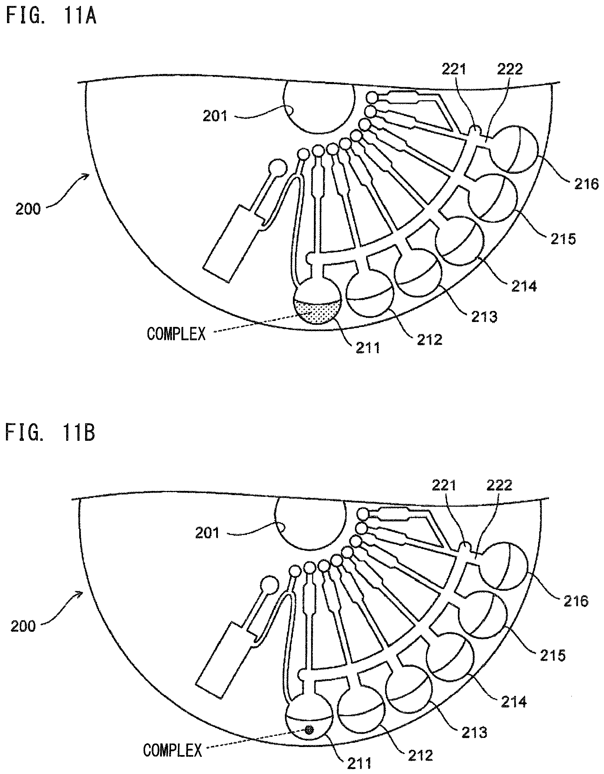

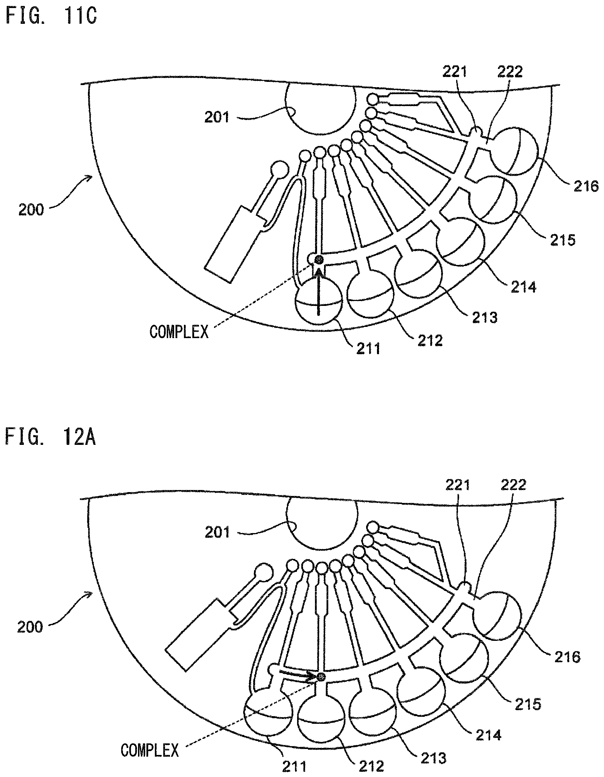

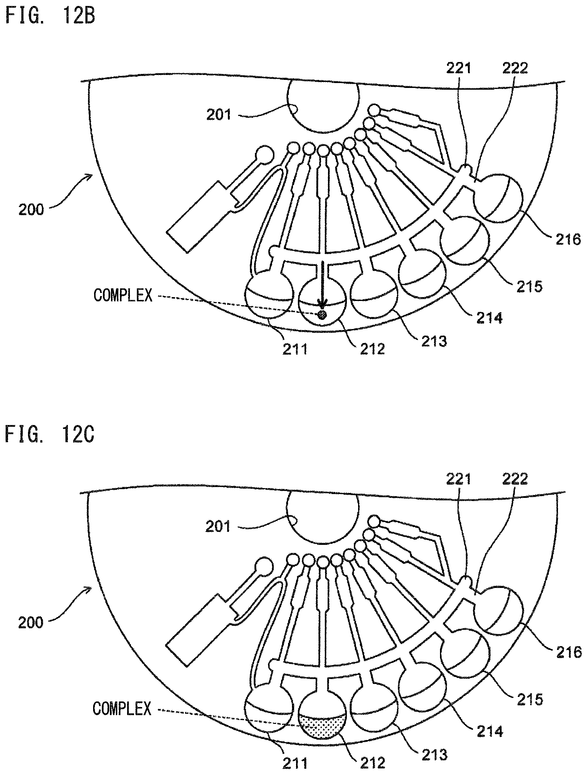

The process in step S 13 will be described in detail with reference to FIG. 10. FIG. 10 shows a flowchart for explaining step S13 in FIG. 9 in detail. In the following description, FIG. 10 is mainly referred to, and FIGS. 11 and 12 are referred to as appropriate.

At the time when the process of step S12 is finished, the complex disperses in the liquid in the chamber 211 as shown in FIG. 11A. In step S101, the controller 301 drives the movement mechanism 130 to bring the magnet 120 close to the cartridge 200, thereby collecting the complex dispersing in the chamber 211, as shown in FIG. 11B. At this time, the controller 301 causes the tip portion 122a of the magnet 120 to approach a region that is at the center of the chamber 211 in the circumferential direction and is near the outer edge of the chamber 211 in the radial direction, as viewed in a horizontal plane.

In step S102, the controller 301 drives the movement mechanism 130 to move the magnet 120 in a direction approaching the rotation shaft 311, thereby transferring the complex to a connection portion between the region 221 and the region 222 that is connected to the chamber 211, as shown in FIG. 11C. The speed at which the complex is moved with respect to the cartridge 200 in step S102 is set to a speed that is determined in advance so as to prevent the complex from being left in the chamber 211. This speed is preferably not higher than 10 mm/sec, and for example, is set to 0.5 mm/sec.

In step S103, the controller 301 drives the motor 171 to rotate the cartridge 200, thereby transferring the complex to a connection portion between the region 221 and the region 222 that is connected to the chamber 212, as shown in FIG. 12A. The speed at which the complex is moved with respect to the cartridge 200 in step S103 is set to the same speed as in step S102.

In step S104, the controller 301 drives the movement mechanism 130 to move the magnet 120 in a direction away from the rotation shaft 311, thereby transferring the complex to the chamber 212 as shown in FIG. 12B. The speed at which the complex is moved with respect to the cartridge 200 in step S104 is set to the same speed as in step S102. In step S105, the controller 301 drives the movement mechanism 130 to move the magnet 120 away from the cartridge 200, thereby causing the complex to be dispersed in the chamber 212 as shown in FIG. 12C.

As described above, in steps S101 to S105, the controller 301 causes the magnet 120 to move close to the cartridge 200 at the position opposed to the chamber 211, and then causes the magnet 120 to move along the channel 220 while keeping the magnet 120 close to the cartridge 200, thereby locating the magnet 120 at the position opposed to the chamber 212. Thereafter, the controller 301 causes the magnet 120 to move away from the cartridge 200, thereby releasing the complex from magnetic attraction by the magnet 120.

In step S106, the controller 301 causes the agitation process described above to be performed. At this time, since the complex has been released from magnetic attraction before the agitation process and the complex disperses in the chamber 212, agitation of the liquid in the chamber 212 is reliably performed.

The process in step S 13 shown in FIG. 9 is performed as described above. The transfer process and the agitation process shown in steps S101 to S106 are similarly performed also in each of steps S 14 to S 17 described later.

Returning to FIG. 9, in step S14, the controller 301 causes the complex in the chamber 212 to be transferred from the chamber 212 to the chamber 213. Thereby, the complex formed in the chamber 212 and the washing liquid are mixed together in the chamber 213. When the agitation process is performed in step S14, the complex and unreacted substances are separated from each other in the chamber 213. That is, in the chamber 213, unreacted free ingredients are removed by washing.

In step S15, the controller 301 causes the complex in the chamber 213 to be transferred from the chamber 213 to the chamber 214. Thereby, the complex formed in the chamber 212 and the washing liquid are mixed together in the chamber 214. Also in the chamber 214, unreacted free ingredients are removed by washing.

In step S16, the controller 301 causes the complex in the chamber 214 to be transferred from the chamber 214 to the chamber 215. Thereby, the complex formed in the chamber 212 and the washing liquid are mixed together in the chamber 215. Also in the chamber 215, unreacted free ingredients are removed by washing.

In step S 17, the controller 301 causes the complex in the chamber 215 to be transferred from the chamber 215 to the chamber 216. Thereby, the complex formed in the chamber 212 and the R4 reagent are mixed together in the chamber 216. When the agitation process is performed in step S 17, the complex formed in the chamber 212 is dispersed.

In step S18, the controller 301 causes the R5 reagent to be transferred to the chamber 216. Specifically, the controller 301 drives the motor 171 to move the cartridge 200 in the circumferential direction, and drives the seal opening unit 195 to press down the seal 232a located at the position opposed to the seal opening unit 195. Then, the controller 301 drives the motor 171 to rotate the cartridge 200, thereby transferring, by a centrifugal force, the R5 reagent stored in the storage portion 232 to the chamber 216. Thus, in the chamber 216, the R5 reagent is further mixed with the liquid mixture generated in step S 17.

When the liquid mixture generated in step S 17 and the R5 reagent have been mixed together and subjected to the agitation process in step S18, a measurement sample is prepared. In this measurement sample, chemiluminescence occurs as a signal due to reaction between the labeling substance (alkaline phosphatase) in the complex and the chemiluminescent substrate.

In step S 19, the controller 301 drives the motor 171 to locate the chamber 216 at the position directly above the photodetector 144a, and causes the photodetector 144a to detect light generated from the chamber 216. In step S20, the controller 301 performs an analysis process regarding immunity, on the basis of the light detected by the photodetector 144a. The controller 301 analyzes presence/absence of the test substance, the amount of the test substance, and the like, on the basis of an output from the light detection unit 144, and causes a display unit (not shown) to display analysis results. The display unit is provided on, for example, the side surface portion of the body 101 or the upper surface portion of the lid 102. The display unit is implemented by a liquid crystal panel or the like.

In the example described above, a second capture substance labeled with a fluorescent substance may be used. In this case, a light source for irradiating the chamber 216 with excitation light is provided. The photodetector 144a detects fluorescence that is emitted from the fluorescent substance in the complex due to the light irradiation from the light source.

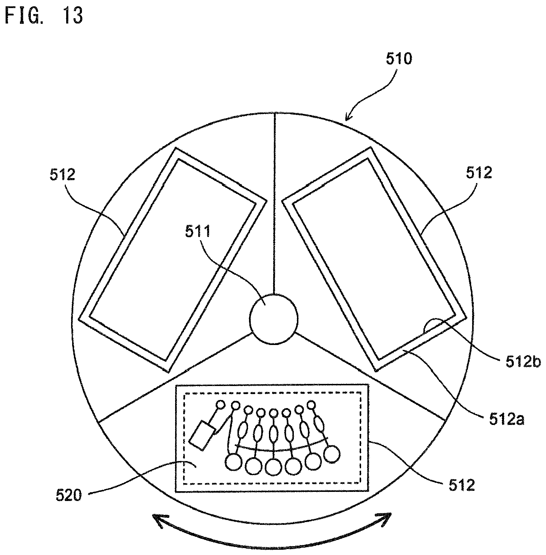

In another embodiment, as shown in FIG. 13, a support member 510 is provided instead of the support member 177, and a rectangular cartridge 520 is used instead of the disk-shaped cartridge 200. Other components are the same as the specific components of the analyzer 100 described above.

The support member 510 includes a hole 511, and three mounting portions 512. The hole 511 is provided at the center of the support member 510. The support member 510 is mounted to the rotation shaft 311 via a predetermined member. Thereby, the support member 510 is rotatable around the rotation shaft 311. The three mounting portions 512 are provided in the circumferential direction. Each mounting portion 512 has a surface 512a and a hole 512b. The surface 512a is one level lower than an upper surface of the support member 510. The hole 512b is formed at the center of the surface 512a, and penetrates the support member 510 in the up-down direction. The cartridge 520 has a structure similar to that of the cartridge 200 except that it is rectangular in shape.

When analysis is started, an operator infuses the sample into the cartridge 520 and sets the cartridge 520 on the mounting portion 512, as in the case of the cartridge 200. Then, as described above, the controller 301 drives the motor 171, the movement mechanism 130, and the detection unit 140. Thereby, transfer of the complex in the cartridge 520 is reliably performed by the magnet 120. Therefore, accuracy of analysis of the test substance by the analyzer 100 can be kept high. Further, in this embodiment, since the cartridge 520 can be set on each of the three mounting portions 512, three cartridges 520 can be subjected to analysis at a time.

### [4. Method for immobilizing carrier particles]

A method for immobilizing carrier particles onto a solid phase (hereinafter also simply referred to as "immobilization method") is also within the scope of the present disclosure. According to the immobilization method of the present embodiment, carrier particles can be immobilized onto any solid phase by means of a solid mixture containing sugar and protein. In the immobilization method of the present embodiment, first, carrier particles are dispersed into a solution containing sugar and protein to obtain a suspension of the carrier particles. Details such as the composition of the suspension of the carrier particles are the same as those described for the cartridge production method of the present embodiment.

Next, the suspension is supplied to a solid phase. In the present embodiment, the solid phase is not particularly limited, and may be selected from among solid phases generally used for immunological measurement. Examples of the solid phase include a micro-plate, a micro-tube, and a test tube. The solid phase may be a microfluidic device such as the cartridge base body described above. The material of the solid phase is not particularly limited, but is preferably the same as the material of the cartridge base body described above. The supplied amount of the suspension is not particularly limited, and may be determined as appropriate according to the kind of the solid phase.

Then, the supplied suspension is dried, and the carrier particles are immobilized onto the solid phase. Details such as means and conditions for drying are the same as those described for the cartridge production method of the present embodiment. After the drying, the carrier particles are immobilized onto the solid phase by the solid mixture containing sugar and protein. The carrier particles are firmly immobilized onto the solid phase, but are easily dispersed into a solution when coming into contact with the solution.

Various modifications of the present disclosure may be attained other than the above mentioned embodiments. Such modifications should not be deemed to be out of the scope of the present disclosure. The present disclosure should include all the modifications within the scope of the claims, their equivalents, and within the above scope.

Hereinafter, the present disclosure will be described in detail with reference to Examples. However, the present disclosure is not limited to these Examples. In the following description, "HISCL" is a registered trademark of Sysmex Corporation. In addition, "mass%" is represented as "wt%".

### EXAMPLES

### Example 1: Evaluation of performance of a cartridge for sample analysis to which carrier particles are immobilized

In Example 1, immobilization strength and dispersibility of magnetic particles dried and immobilized to a chamber of a cartridge for sample analysis were evaluated.

### (1) Material

### (1.1) Cartridge base body

In Example 1, a micro flow path cartridge base body made of polymethyl methacrylate (ASTI Corporation) was used. This cartridge base body includes an opening through which a liquid sample is infused, a sample storage portion, a plurality of chambers, and a flow path connecting them. The volume of each chamber is about 34 µL, and the inner diameter of the flow path is about 2 µm.

### (1.2) Reagent containing magnetic particles

Streptavidin-bound magnetic particles (HISCL R2 reagent: Sysmex Corporation) were suspended in a water solution containing sugar and protein (1 wt% BSA, 2.5 wt% sucrose, and 20 mM MES (pH 6.5)) to prepare Reagent 1 containing the magnetic particles (particle concentration = 1 wt%, mass ratio of sugar to protein [sugar/protein] = 2.5) (hereinafter also referred to as "Reagent 1 ").

### (1.3) Eluting reagent

A calibrator (HISCL TSH C0, TSH concentration = 0 IU/mL) contained in an HISCL TSH reagent (Sysmex Corporation) as a thyroid stimulating hormone measurement kit was mixed with an HISCL TSH R3 reagent at a ratio of 1:1 (volume ratio) to prepare an eluting reagent. In Example 1, this eluting reagent was used for confirming dispersibility of the magnetic particles dried and immobilized to the inner wall of the chamber. The HISCL TSH R3 reagent is a water solution containing a biotin-bound anti-TSH monoclonal antibody.

### (1.4) Sample analyzer

A prototype of a sample analyzer (Sysmex Corporation) capable of centrifuging the cartridge base body of above (1.1) was used (hereinafter, this prototype is also referred to as "analyzer").

### (2) Production of cartridge for sample analysis

Reagent 1 (10 µL) was added to a reaction chamber of a cartridge. This cartridge was put in a digital control desiccator (McDRY MCU-201: ERC Co., Ltd.), and was dried for 1 hour at room temperature. Through the drying process, Reagent 1 was dried and solidified in the chamber, and magnetic particles were immobilized to the inner wall of the chamber by means of a solid mixture containing sugar and protein. Thus, a cartridge for sample analysis was obtained. A plurality of cartridges for sample analysis were produced in the same manner as described above.

### (3) Evaluation of external appearance, immobilization strength, and dispersibility of dried and immobilized magnetic particles

### (3.1) External appearance and immobilization strength

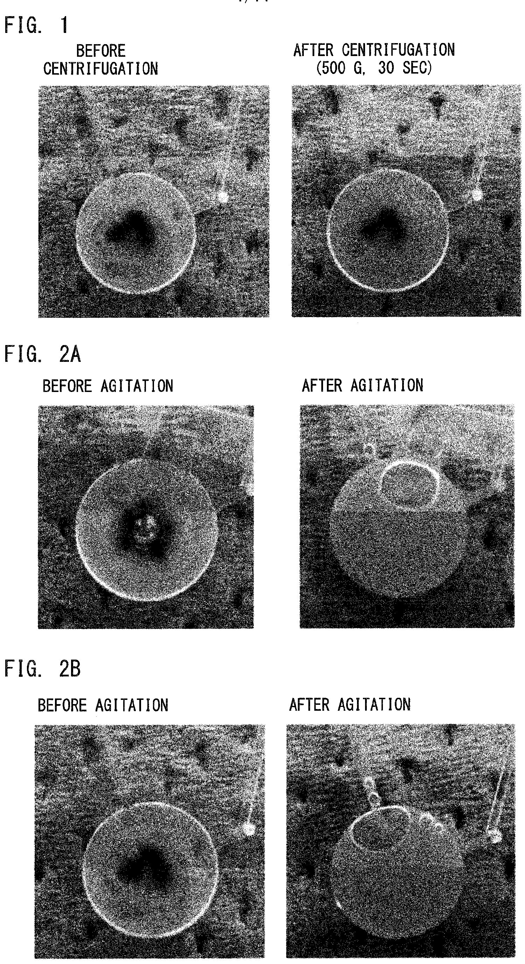

The surfaces of the dried and immobilized magnetic particles were observed to confirm presence/absence of cracks. The cartridge to which the magnetic particles were immobilized was horizontally set in the analyzer, and was centrifuged for 30 seconds at 3000 rpm or for 10 seconds at 6000 rpm around a rotation shaft of the analyzer. Thereby, a centrifugal force (500 G or 2000 G) was applied to the magnetic particles immobilized to the chamber. FIG. 1 shows photographs of the chamber before and after application of the centrifugal force of 500 G. Meanwhile, another cartridge was lightly hit against a desk to apply impact to the magnetic particles immobilized to the chamber.

As seen from the left photograph in FIG. 1, before centrifugation, the magnetic particles were immobilized onto the inner wall of the round chamber. No crack was observed in the magnetic particles immobilized to the chamber. As seen from the right photograph in FIG. 1, even when the centrifugal force of 500 G was applied, the magnetic particles remained immobilized without moving in the chamber or separating from the chamber. Also when a centrifugal force of 2000 G was applied, movement and separation of the magnetic particles were not observed (not shown). These conditions for centrifugation are the same as the conditions used for separating plasma from blood. Further, the magnetic particles were not separated from the inner wall of the chamber even when impact was applied thereto. Accordingly, it was confirmed that the magnetic particles were firmly immobilized to the chamber in the cartridge of the present embodiment.

### (3.2) Dispersibility

The eluting reagent (20 µL) was infused to the opening of the cartridge to which the magnetic particles were immobilized. Then, this cartridge was centrifuged by the analyzer to agitate the eluting reagent and the magnetic particles. This agitation by centrifugation was performed by repeating, for 30 seconds, acceleration of the rotation speed from 300 rpm to 500 rpm within 0.02 second and deceleration of the rotation speed from 500 rpm to 300 rpm within 0.02 second. The eluting reagent was transferred to the chamber through the flow path by the centrifugal force, and came into contact with the magnetic particles immobilized to the chamber. FIG. 2A shows photographs of the chamber before and after the agitation. Meanwhile, another cartridge was subjected to similar test for dispersibility with conditions for agitation being changed. In this test, agitation was performed by repeating, for 30 seconds, acceleration of the rotation speed from 50 rpm to 250 rpm within 0.02 second and deceleration of the rotation speed from 250 rpm to 50 rpm within 0.02 second. FIG. 2B shows photographs of the chamber before and after the agitation.

As seen from the left photograph in FIG. 2A, before the agitation by centrifugation, the magnetic particles were immobilized to the inner wall of the round chamber. As seen from the right photograph in FIG. 2A, after the agitation, the solid mixture was dissolved due to contact with the eluting reagent, and the magnetic particles were separated from the inner wall. Aggregation of the magnetic particles was not observed in the solution. That is, it is found that the magnetic particles separated from the inner wall were dispersed into the solution. As shown in FIG. 2B, the same results as shown in FIG. 2A were obtained even when the conditions for agitation by centrifugation were changed. From the above, it is confirmed that, in the cartridge of the present embodiment, the magnetic particles immobilized to the chamber can be easily dispersed into the solution containing the sample by agitation using centrifugation.

### Example 2: Evaluation of performance of dried and immobilized carrier particles

In Example 2, influence on immunological measurement by drying and immobilization of magnetic particles, and preservation stability of dried and immobilized magnetic particles were evaluated.

### (1) Material

### (1.1) Reagent containing magnetic particles

Reagent 1 prepared in Example 1 was used as a reagent containing magnetic particles. For comparison, streptavidin-bound magnetic particles were suspended in a water solution (20 mM MES (pH 6.5)) that does not contain sugar and protein, to prepare Reagent 2 containing the magnetic particles (particle concentration = 1 wt%) (hereinafter also referred to as "Reagent 2").

### (1.2) Immobilization of magnetic particles to substrate, and collection thereof

50 µL of reagent 1 was dropped on each of two cycloolefin copolymer (COP) substrates (ASTI Corporation). These substrates were left still in a plastic container, together with calcium chloride desiccant, and air-dried in a hermetically sealed state. Thereby, magnetic particles were dried and immobilized onto the substrates. Purified water (50 µL) was dropped on the magnetic particles immobilized to one of the substrates, and the magnetic particles were collected by pipetting. The other substrate was sealed in an aluminum laminate bag, and preserved in a constant-temperature apparatus at 45°C for three days. Three days later, purified water (50 µL) was dropped on magnetic particles immobilized to the substrate, and the magnetic particles were collected by pipetting. Also regarding Reagent 2, magnetic particles thereof were dried and immobilized to two COP substrates in the same manner as described above, and the magnetic particles were collected by using purified water (50µL) from one of the substrates. Further, the other substrate was preserved at 45°C for three days in the same manner as described above, and thereafter, magnetic particles on the substrate were collected by using purified water (50 µL).

### (2) Immunological measurement

Immunological measurement was performed using the collected magnetic particles and the HISCL TSH reagent (Sysmex Corporation). Immunological measurement was specifically performed as follows.

### (2.1) Sample, reagent, and measurement apparatus

Sample (calibrator): HISCL TSH C0 (TSH concentration = 0 IU/mL), and HISCL TSH C3 (TSH concentration = 50 µIU/mL) (Sysmex Corporation)
First reagent (capture antibody): HISCL TSH R1 reagent (Sysmex Corporation)
Second reagent (carrier particles): a suspension of the magnetic particles collected in above (1.2)
Third reagent (detection antibody): HISCL TSH R3 reagent (Sysmex Corporation)
Washing liquid: HISCL washing liquid (Sysmex Corporation)
Fourth reagent (substrate buffer): HISCL R4 reagent (Sysmex Corporation)
Fifth reagent (luminescent substrate): HISCL R5 reagent (CDP-Star (registered trademark)) (Sysmex Corporation)
Measurement apparatus: full-automatic immunoassay apparatus HISCL-800

### (Sysmex Corporation)

### (2.2) Procedure of measurement

Measurement was performed by using HISCL-800 set by default. However, operation to add the second reagent was performed by a hand method. The sample (30 µL) and the first reagent (30 µL) were added to a reaction cuvette, and incubation was performed at 42°C for 2 minutes. The second reagent (30 µL) was added to the reaction cuvette, and incubation was performed at 42°C for 2 minutes and 30 seconds. The third reagent (30 µL) was added to the reaction cuvette, and incubation was performed at 42°C for 2 minutes and 30 seconds. The magnetic particles were collected by using a magnet, and the supernatant was removed. The HISCL washing liquid (300 µL) was added, and the magnetic particles were washed (B/F separation). Another three times of B/F separation were performed. The supernatant was removed, and the fourth reagent (50 µL) and the fifth reagent (100 µL) were added to the magnetic particles. Thus obtained liquid mixture was incubated at 42°C for 5 minutes, and a signal (luminescence intensity) was measured. As a control, similar measurement was performed by using an HISCL TSH R2 reagent (Sysmex Corporation) which has been refrigerated for 0 day or 3 days, instead of the collected magnetic particles. The HISCL TSH R2 reagent is an aqueous suspension containing streptavidin-bound magnetic particles.

### (3) Results

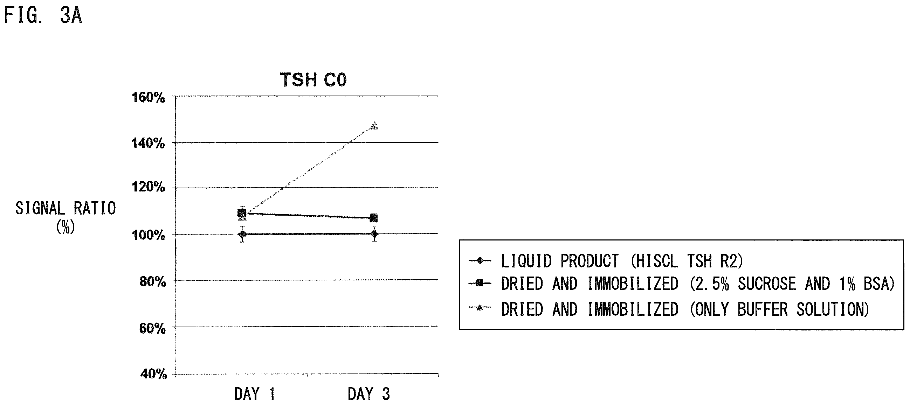

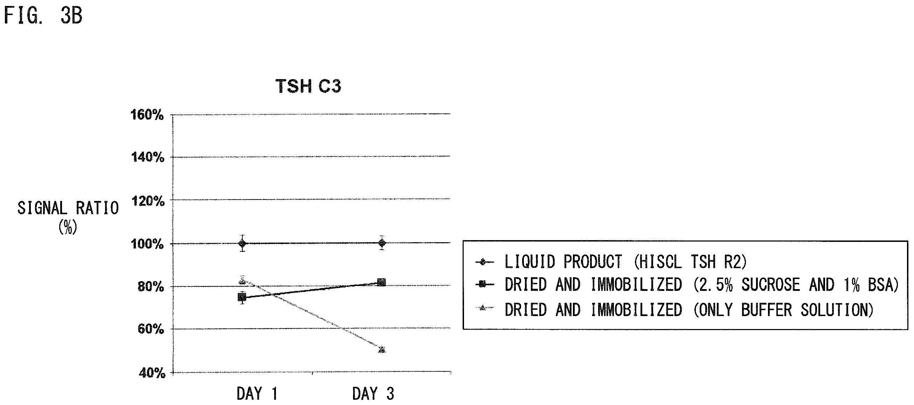

In FIGS. 3A and 3B, the measurement result is represented by the ratio (%) of a signal obtained by measurement using the collected magnetic particles to a signal obtained by measurement using the HISCL TSH R2 reagent. In FIGS. 3A and 3B, "Day 0" indicates the ratio obtained when the magnetic particles before being preserved is used, and "Day 3" indicates the ratio obtained when the magnetic particles preserved for three days are used. In FIG. 3A, since HISCL TSH C0 does not contain TSH as the test substance, the obtained signal indicates background (noise). With reference to FIG. 3A, in Day 0, the signal based on the magnetic particles of Reagent 1 was increased by 7% to 8%, compared to the signal based on the HISCL TSH R2 reagent. Also in Day 3, like in Day 0, the signal based on the magnetic particles of Reagent 1 was increased by about 7%, compared to the signal based on the HISCL TSH R2 reagent. Thus, between Day 0 and Day 3, no remarkable change was observed in the signal based on the magnetic particles of Reagent 1. That is, although the noise was slightly increased due to drying and immobilization of the magnetic particles, the measurement values themselves were hardly affected by drying and immobilization of the magnetic particles. In Day 0, the signal based on the magnetic particles of Reagent 2 was increased by about 7%, compared to the HISCL TSH R2 reagent, like the signal based on the magnetic particles of Reagent 1. However, in Day 3, the signal based on the magnetic particles of Reagent 2 was increased by 40% or more, compared to the HISCL TSH R2 reagent.

In FIG. 3B, since HISCL TSH C3 contains TSH as the test substance, the obtained signal indicates the amount of the detected test substance. With reference to FIG. 3B, in Day 0, the signal based on the magnetic particles of Reagent 1 was reduced by about 20%, compared to the HISCL TSH R2 reagent. In Day 3, like in Day 0, the signal based on the magnetic particles of Reagent 1 was reduced by about 20%, compared to the HISCL TSH R2 reagent. Although the signal was reduced due to drying and immobilization of the magnetic particles, sufficient measurement values were obtained in detection of the test substance. Thus, between Day 0 and Day 3, no remarkable change was observed in the signal based on the magnetic particles of Reagent 1. In Day 0, the signal based on the magnetic particles of Reagent 2 was reduced by about 20%, compared to the HISCL TSH R2 reagent, like the signal based on the magnetic particles of Reagent 1. However, in Day 3, the signal based on the magnetic particles of Reagent 2 was reduced by about 50%, compared to the HISCL TSH R2 reagent.

From the above, it was suggested that, when the magnetic particles are dried and immobilized without sugar and protein, the magnetic particles cannot be stably preserved. In contrast, when the magnetic particles are dried and immobilized with sugar and protein, the magnetic particles are stably preserved.

### Example 3: Examination on sugar concentration

In Example 3, an appropriate concentration of sugar in a reagent containing magnetic particles to be dried and immobilized to the cartridge for sample analysis was examined on the basis of dispersibility of the magnetic particles after being dried.

### (1) Material

### (1.1) Reagent containing magnetic particles

Reagents 3 to 5 each containing magnetic particles were prepared as follows.

### Reagent 3 containing magnetic particles

Streptavidin-bound magnetic particles (HISCL R2 reagent: Sysmex Corporation) were suspended in a water solution containing sugar and protein (1 wt% BSA, 0.5 wt% trehalose, and 20 mM MES (pH 6.5)) to prepare Reagent 3 containing the magnetic particles (particle concentration = 1 wt%, [sugar/protein] = 0.5) (hereinafter also referred to as "Reagent 3").

### Reagent 4 containing magnetic particles

Streptavidin-bound magnetic particles were suspended in another water solution containing sugar and protein (1 wt% BSA, 0.1 wt% trehalose, and 20 mM MES (pH 6.5)) to prepare Reagent 4 containing magnetic particles (particle concentration = 1 wt%, [sugar/protein] = 0.1) (hereinafter also referred to as "Reagent 4").

### Reagent 5 containing magnetic particles

Streptavidin-bound magnetic particles were suspended in still another water solution containing sugar and protein (0.35 wt% BSA, 35.4 wt% trehalose, and 20 mM MES (pH 6.5)) to prepare Reagent 5 containing the magnetic particles (particle concentration = 1 wt%, [sugar/protein] = 101.1) (hereinafter also referred to as "Reagent 5").

### (1.2) Immobilization of magnetic particles to cartridge

Reagent 3 (10µL) was added into a reaction chamber of the same cartridge base body as described for Example 1. This cartridge base body was left still in a plastic container together with calcium chloride desiccant, and air-dried in a hermetically sealed state. Thus, a cartridge in which the magnetic particles of Reagent 3 were dried and immobilized was obtained. Likewise, a cartridge in which the magnetic particles of Reagent 4 were dried and immobilized was produced. The cartridges produced were lightly hit against a desk to confirm that the magnetic particles were firmly immobilized to the inner wall of the chamber.

Meanwhile, the cartridge base body to which Reagent 5 was added was left still in a container overnight, together with desiccant in the same manner as described above. However, Reagent 5 was not dried, and a large amount of moisture was left therein. That is, a gel substance containing magnetic particles was adhered to the reaction chamber of the cartridge base body. When this cartridge base body was centrifuged at 1000 rpm (60 G), the gel substance was shifted on the inner wall due to the centrifugal force, which means that the immobilization strength was insufficient. As a result, the magnetic particles of the Reagent 5 were not dried and immobilized to the reaction chamber.

### (2) Evaluation of dispersibility

The same eluting reagent (20 µL) as that used in Example 1 was infused to the opening of the cartridge. Then, this cartridge was centrifuged by the analyzer of Example 1 to agitate the eluting reagent and the magnetic particles. This agitation by centrifugation was performed by repeating, for 30 seconds, acceleration of the rotation speed from 50 rpm to 250 rpm within 0.02 second and deceleration of the rotation speed from 250 rpm to 50 rpm within 0.02 second. This agitation operation was performed three times in total. The magnetic particles of Reagent 3 were separated from the inner wall due to contact with the eluting reagent. Aggregation of the magnetic particles was not observed in the solution. Therefore, it was found that the magnetic particles of Reagent 3 were dispersed into the solution. Meanwhile, part of the magnetic particles of Reagent 4 still remained on the inner wall even after contact with the eluting reagent. Further, deposition of the magnetic particles in the solution was observed.

From the above, it was found that, when the concentration of sugar in the reagent containing the magnetic particles is too low, it is difficult to cause the dried and immobilized magnetic particles to be dispersed. On the other hand, it was found that, when the concentration of sugar in the reagent containing the magnetic particles is too high, the magnetic particles are not dried and immobilized. Therefore, it was suggested that the concentration of sugar in the reagent containing the magnetic particles is preferably not less than 0.5 wt% and not greater than 30 wt%.

### Example 4: Examination on protein concentration

In Example 4, an appropriate concentration of protein in a reagent containing magnetic particles to be dried and immobilized to the cartridge for sample analysis was examined on the basis of dispersibility of the magnetic particles after being dried.

### (1) Material

### (1.1) Reagent containing magnetic particles

Streptavidin-bound magnetic particles (HISCL R2 reagent: Sysmex Corporation) were suspend in a water solution containing sugar and protein (0.35 wt% BSA, 3.49 wt% glucose, and 20 mM MES (pH 6.5)) to prepare Reagent 6 containing the magnetic particles (particle concentration = 1 wt%, [sugar/protein] = 9.9) (hereinafter also referred to as "Reagent 6"). For comparison, streptavidin-bound magnetic particles were suspended in another water solution containing sugar and protein (0.04 wt% BSA, 4.14 wt% glucose, and 20 mM MES (pH 6.5)) to prepare Reagent 7 containing the magnetic particles (particle concentration = 1 wt%, [sugar/protein] = 103.5) (hereinafter also referred to as "Reagent 7").

### (1.2) Immobilization of magnetic particles to cartridge

Reagent 6 (10µL) was added into a reaction chamber of the same cartridge base body as described for Example 1. This cartridge base body was left still in a plastic container together with calcium chloride desiccant, and air-dried in a hermetically sealed state. Thus, a cartridge in which the magnetic particles of Reagent 6 were dried and immobilized was obtained. Likewise, a cartridge in which the magnetic particles of Reagent 7 were dried and immobilized was produced. The cartridges produced were lightly hit against a desk to confirm that the magnetic particles were firmly immobilized to the inner wall of the chamber.

### (2) Evaluation of dispersibility

The same eluting reagent (20 µL) as that used in Example 1 was infused to the opening of the cartridge. Then, this cartridge was centrifuged by the analyzer of Example 1 to agitate the eluting reagent and the magnetic particles. The agitation conditions were the same as those in Example 3. The magnetic particles of Reagent 6 were separated from the inner wall due to contact with the eluting reagent. Aggregation of the magnetic particles was not observed in the solution. Therefore, it was found that the magnetic particles of Reagent 6 were dispersed into the solution. Meanwhile, the magnetic particles of Reagent 7 were separated from the inner wall due to contact with the eluting reagent, but deposition of the magnetic particles was observed in the solution. From the above, it was found that, when the concentration of protein in the reagent containing the magnetic particles is too low, it is difficult to cause the dried and immobilized magnetic particles to be uniformly dispersed. As a result, the present inventors considered that the concentration of protein in the reagent containing the magnetic particles is preferably not less than 0.1 wt%.

### Example 5: Examination on ratio of sugar concentration to protein concentration

In Example 5, an appropriate concentration ratio of sugar to protein in a reagent containing magnetic particles to be dried and immobilized to the cartridge for sample analysis was examined on the basis of external appearance and immobilization strength of the magnetic particles after being dried.

### (1) Material

### (1.1) Reagent containing magnetic particles

Reagent 8 to 10 each containing magnetic particles were prepared as follows.

### Reagent 8 containing magnetic particles

Streptavidin-bound magnetic particles (HISCL R2 reagent: Sysmex Corporation) were suspended in a water solution containing sugar and protein (3 wt% BSA, 1.5 wt% trehalose, and 20 mM MES (pH 6.5)) to prepare Reagent 8 containing the magnetic particles (particle concentration = 1 wt%, [sugar/protein] = 0.5) (hereinafter also referred to as "Reagent 8").

### Reagent 9 containing magnetic particles

Streptavidin-bound magnetic particles were suspended in another water solution containing sugar and protein (15.0 wt% BSA, 1.5 wt% glucose, and 20 mM MES (pH 6.5)) to prepare Reagent 9 containing the magnetic particles (particle concentration = 1 wt%, [sugar/protein] = 0.1) (hereinafter also referred to as "Reagent 9").

### Reagent 10 containing magnetic particles

Streptavidin-bound magnetic particles were suspended in still another water solution containing sugar and protein (1.9 wt% BSA, 0.19 wt% trehalose, and 20 mM MES (pH 6.5)) to prepare Reagent 10 containing the magnetic particles (particle concentration = 1 wt%, [sugar/protein] = 0.1) (hereinafter also referred to as "Reagent 10").

### (1.2) Immobilization of magnetic particles to cartridge

Reagent 8 (10µL) was added into a reaction chamber of the same cartridge base body as described for Example 1. The cartridge base body was left still in a plastic container together with calcium chloride desiccant, and air-dried in a hermetically sealed state. Thus, a cartridge in which the magnetic particles of Reagent 8 were dried and immobilized was obtained. Likewise, cartridges in which the magnetic particles of Reagent 9 and the magnetic particles of Reagent 10 were dried and immobilized were produced, respectively.

### (2) Evaluation of external appearance and immobilization strength of dried and immobilized magnetic particles

No crack was observed in the dried and immobilized magnetic particles of Reagent 8. Even when the cartridge to which the magnetic particles of Reagent 8 were immobilized was centrifuged at 1000 rpm (60 G), the magnetic particles did not move and separate from the inner wall of the chamber. On the other hand, cracks were observed in the dried and immobilized magnetic particles of Reagent 9. Such cracks may cause the magnetic particles to be separated from the chamber when vibration or impact is applied during transport of the product. Although no crack was observed in the dried and immobilized magnetic particles of Reagent 10, the magnetic particles were separated from the inner wall of the chamber when the cartridge was centrifuged at 1000 rpm (60 G). From the above, it was found that, when the ratio of the sugar concentration to the protein concentration (sugar concentration /protein concentration) in the reagent containing the magnetic particles is too low, it is difficult to firmly immobilize the magnetic particles to the inner wall of the chamber. Further, it was found that the ratio of the mass of sugar to the mass of protein (sugar/protein) in the reagent containing the magnetic particles is preferably not less than 0.5.

### Comparative Example 1

In Comparative Example 1, a reagent containing magnetic particles, which does not contain sugar and protein, was dried and immobilized to a cartridge, and dispersibility of the immobilized magnetic particles was confirmed. Details are as follows.

### (1) Immobilization of magnetic particles to cartridge

Streptavidin-bound magnetic particles (HISCL R2 reagent: Sysmex Corporation) were suspended in a water solution (20 mM MES (pH 6.5)) that does not contain sugar and protein, to prepare Reagent 11 containing the magnetic particles (particle concentration = 1 wt%) (hereinafter referred to as "Reagent 11 "). The Reagent 11 (10 µL) was added to a reaction chamber of the same cartridge base body as described for Example 1. This cartridge base body was left still in a plastic container together with calcium chloride desiccant, and air-dried in a hermetically sealed state. Thus, a cartridge in which the magnetic particles of Reagent 11 were dried and immobilized was obtained. No crack was observed in the dried and immobilized magnetic particles of Reagent 11.

### (2) Evaluation of dispersibility

The same eluting reagent (20 µL) as that used in Example 1 was infused to the opening of the cartridge. Then, this cartridge was centrifuged by the analyzer of Example 1 to agitate the eluting reagent and the magnetic particles. The agitation conditions were the same as those of Example 3. The agitation operation was performed four times in total, but the magnetic particles of Reagent 11 were hardly separated from the chamber and were kept immobilized to the chamber.

### Comparative Example 2

In Comparative Example 2, a reagent containing magnetic particles, which includes one of sugar and protein, was dried and immobilize onto a substrate, and immobilization strength and dispersibility of the magnetic particles were confirmed. Details are as follows.

### (1) Preparation of reagent, and immobilization to substrate

To a buffer solution (20 mM MES (pH 6.5)), sucrose was added so as to have concentrations of 2, 5, and 10 wt%, thereby preparing water solutions containing the sugar. Parts of the respective water solutions were collected, and magnetic particles were added thereto so as to have a particle concentration of 1 wt%, thereby preparing reagents containing the magnetic particles and the sugar. To a buffer solution (20 mM MES (pH 6.5)), BSA was added so as to have concentrations of 1 and 3 wt%, thereby preparing water solutions containing the protein. Parts of the respective water solutions were collected, and magnetic particles were added thereto so as to have a particle concentration of 1 wt%, thereby preparing reagents containing the magnetic particles and the protein. Each of the reagents containing the magnetic particles was dropped in an amount of 10 µL onto a PMMA substrate (ASTI Corporation) and a COP substrate (ASTI Corporation). These substrates were left still in a plastic container together with calcium chloride desiccant, and air-dried in a hermetically sealed state. Thereby, the magnetic particles were dried and immobilized onto the substrates.

### (2) Evaluation of immobilization strength and dispersibility of magnetic particles

The surfaces of the magnetic particles on the respective substrates were observed to confirm whether cracks occurred. Then, the substrates were lightly hit against a desk to apply impact to the immobilized magnetic particles, and it was confirmed whether the magnetic particles were separated from the substrates. Meanwhile, the same eluting reagent (10 µL) as that used in Example 1 was dropped on magnetic particles immobilized to other substrates, and dispersibility of the magnetic particles was confirmed. Results are shown in Table 1. In Table 1, in the column of "immobilization strength", "○" indicates that the magnetic particles were not separated from the substrate even when impact was applied, and "×" indicates that the magnetic particles were separated from the substrate due to impact. In the column of "dispersibility", "○" indicates that the magnetic particles were dispersed into the eluting reagent, and "×" indicates that the magnetic particles were not sufficiently dispersed into the eluting reagent.

**Table 1**

| No. | Substrate | Composition of reagent before being dried | Crack | Immobilization Strength | Dispersibility |
|---|---|---|---|---|---|
| 1 | PMMA | 1% magnetic particles, 2% sucrose, and 20 mM MES (pH 6.5) | Absent | ○ | × |
| 2 | PMMA | 1% magnetic particles, 5% sucrose, and 20 mM MES (pH 6.5) | Absent | ○ | × |
| 3 | PMMA | 1% magnetic particles, 10% sucrose, and 20 mM MES (pH 6.5) | Absent | ○ | × |
| 4 | PMMA | 1% magnetic particles, 1% BSA, and 20 mM MES (pH 6.5) | Present | × | ○ |
| 5 | PMMA | 1% magnetic particles, 3% BSA, and 20 mM MES (pH 6.5) | Present | × | ○ |
| 6 | COP | 1 % magnetic particles, 2% sucrose, and 20 mM MES (pH 6.5) | Absent | ○ | × |
| 7 | COP | 1 % magnetic particles, 5% sucrose, and 20 mM MES (pH 6.5) | Absent | ○ | × |
| 8 | COP | 1% magnetic particles, 10% sucrose, and 20 mM MES (pH 6.5) | Absent | ○ | × |
| 9 | COP | 1 % magnetic particles, 1% BSA, and 20 mM MES (pH 6.5) | Present | × | ○ |
| 10 | COP | 1% magnetic particles, 3% BSA, and 20 mM MES (pH 6.5) | Present | × | ○ |

As seen from Table 1, when the reagents containing no protein (Nos. 1-3 and Nos. 6-8) were dried and immobilized, the magnetic particles thereof were firmly immobilized onto the substrates without generating cracks. However, dispersibility of the magnetic particles was not satisfactory. Therefore, the magnetic particles of these reagents were not suitable for sample analysis. On the other hand, when the reagents containing no sugar (Nos. 4, 5, 9, and 10) were dried and immobilized, cracks occurred on the surfaces of the magnetic particles. In addition, the magnetic particles were separated from the substrate due to impact. Therefore, the magnetic particles of these reagents may not bear vibration and/or impact during transport. From the above, it was found that both immobilization strength and dispersibility cannot be satisfied by only one of sugar and protein.

### DESCRIPTION OF THE REFERENCE CHARACTERS

100 analyzer
101 body
101a, 102a casing
102 lid
110,180 mounting member
111,112,113,114 hole
115, 116 protruding portion
117, 182 elastic member
120 magnet
121 permanent magnet
122 magnetic substance
122a tip portion
130 movement mechanism
131 attachment member
131 a, 134a hole
134,143 support portion
135, 136 motor
140 detection unit
142 reflection member
144 light detection unit
144a photodetector
150 housing
151 upper surface
152, 153 storage portion
155 hole
160 light adjustment unit
162a hole
162c filter member
171 motor
171a drive shaft
172 encoder
176,191 plate member
177 support member
181 protruding portion
192 clamper
193 image capturing unit
194 lighting unit
195 seal opening unit
200 cartridge
200a base body
201 hole
211 to 216 chamber
220,243 channel
221,222 region
231, 232 storage portion
231a, 232a seal
241 opening
242 separator
243a hole
243b region
301 controller
310 mounting member
311 rotation shaft
312 fixing member
321,322 heater
340 dark space
510 support member
520 cartridge

## Claims

1. A cartridge for sample analysis, comprising:
a cartridge base body having, formed therein, a chamber configured to store a liquid containing a test substance, and a storage portion which is connectable with the chamber and is configured to store a liquid reagent to be supplied to the chamber;
carrier particles immobilized onto an inner wall of the chamber;
a first liquid reagent stored in the storage portion; and
a first capture substance that specifically binds to the test substance, wherein
the carrier particles are immobilized onto the inner wall by means of a solid mixture containing sugar and protein.

2. The cartridge of claim 1, wherein a mass ratio of the sugar to the protein in the solid mixture is not less than 0.1 and not greater than 100.

3. The cartridge of claim 2, wherein the mass ratio of the sugar to the protein in the solid mixture is not less than 0.5 and not greater than 30.

4. The cartridge of any one of claims 1 to 3, wherein a mass ratio of the carrier particles to the protein contained in the solid mixture is not less than 0.05 and not greater than 10.

5. The cartridge of any one of claims 1 to 4, wherein
the carrier particles are obtained by drying, on the inner wall of the chamber, a suspension of the carrier particles which is obtained by dispersing the carrier particles into the solution containing sugar and protein, and
a concentration of the protein in the suspension of the carrier particles is not less than 0.1 mass% and not greater than 20 mass%.

6. The cartridge of claim 5, wherein a concentration of the sugar in the suspension of the carrier particles is not less than 0.5 mass% and not greater than 30 mass%.

7. The cartridge of any one of claims 1 to 6, wherein the first capture substance is contained in the first liquid reagent.

8. The cartridge of any one of claims 1 to 6, wherein the first capture substance is immobilized to surfaces of the carrier particles.

9. The cartridge of any one of claims 1 to 8, wherein the first capture substance is an antibody or an antigen that specifically binds to the test substance.

10. The cartridge of any one of claims 1 to 9, wherein the carrier particles are magnetic particles.

11. The cartridge of any one of claims 1 to 10, wherein the sugar contained in the solid mixture is at least one selected from among a monosaccharide and a disaccharide.

12. The cartridge of claim 11, wherein the sugar contained in the solid mixture is at least one selected from among sucrose, trehalose, glucose, lactose, fructose, maltose, and galactose.

13. The cartridge of any one of claims 1 to 12, wherein the protein contained in the solid mixture is at least one selected from among proteins having function of stabilizing the carrier particles and the first capture substance.

14. A method for detecting a test substance by using a cartridge for sample analysis, comprising:
bringing carrier particles immobilized to an inner wall of a chamber formed in the cartridge for sample analysis, a sample containing the test substance, a first capture substance that specifically binds to the test substance, and a first liquid reagent, into contact with each other in the chamber;
dispersing, by agitation, the carrier particles into a liquid mixture containing the test substance, the first capture substance, and the first liquid reagent;
forming a complex containing the test substance and the first capture substance, on the dispersed carrier particles; and
detecting the test substance contained in the complex, wherein
in the cartridge for sample analysis, the carrier particles are immobilized onto the inner wall by means of a solid mixture containing sugar and protein.

15. A method for immobilizing carrier particles, comprising:
dispersing the carrier particles into a solution containing sugar and protein to obtain a suspension of the carrier particles;
supplying the suspension onto a solid phase; and
drying the supplied suspension to immobilize the carrier particles onto the solid phase.
